# EUROPEAN PATENT APPLICATION

(11) **EP 4 775 560 A1**
(43) Date of publication of application: **15.07.2026**
(21) Application number: 24861760.7
(22) Date of filing: 08.08.2024
(51) Int. Cl.: C07C 5/27, C07C 4/06, C10G 65/10

(54) **CHEMICAL CONVERSION METHOD FOR HYDROCARBON STREAM**

(30) Priority: 04.09.2023 CN 202311126558; 04.09.2023 CN 202311126315
(71) Applicant: China Petroleum & Chemical Corporation, Beijing 100728 (CN); Sinopec Dalian Research Institute of Petroleum and Petrochemicals Co., Ltd., Lushunkou District Dalian, Liaoning 116045 (CN)
(72) Inventor: CUI, Zhe, Dalian, Liaoning 116045 (CN); DU, Yanze, Dalian, Liaoning 116045 (CN); ZENG, Ronghui, Dalian, Liaoning 116045 (CN); PENG, Shaozhong, Dalian, Liaoning 116045 (CN); LIU, Chang, Dalian, Liaoning 116045 (CN); WU, Ziming, Dalian, Liaoning 116045 (CN); HAO, Wenyue, Dalian, Liaoning 116045 (CN)
(74) Representative: karo IP
(86) International application number: PCT/CN2024/110543
(87) International publication number: WO 2025/050915

(57) **Abstract**

The present invention relates to a process for the chemical conversion of a hydrocarbon stream. The chemical conversion process of the present invention comprises the steps of: providing a hydrocarbon stream comprising a paraffinic alkane represented by the following structural formula (I), converting at least a part of the paraffinic alkanes in a conversion reaction to C₂-C₆ n-alkanes in the presence of a catalyst and hydrogen gas to obtain a converted product, wherein the catalyst comprises a molecular sieve and a non-noble metal hydrogenation active metal component, and steam cracking the converted product to obtain a cracked product comprising ethylene. 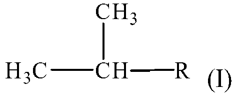 in the structural formula (I), R is C₂-C₆ linear or branched alkyl.

According to the invention, low-quality ethylene raw materials can be effectively converted into high-quality ethylene raw materials, the yield of ethylene and triene of an ethylene device can be greatly improved by the hydrocarbon stream after hydrogenation conversion, the process is simple, the operation difficulty is low, and the process has good economic benefits.

## Description

### Technical Field

The invention relates to the technical field of petrochemical industry, in particular to a process for chemical conversion of a hydrocarbon stream.

### Background

Both naphtha obtained from distillation of crude oil and light naphtha obtained from cracking reaction (including hydrocracking, catalytic cracking and the like) with a molecular sieve-containing catalyst contain a large amount of 2-methylalkanes (including isopentane, 2-methyl pentane, pentane, 2-methyl hexane and the like) as a part of the small molecule iso-alkanes. The 2-methylalkanes can be used as a blending component for gasoline, but with a limited blending amount due to the influence by the saturated vapor pressure. Meanwhile, with the continuous development of the chemical industry, the productivity of ethylene is also increasing year by year, which results in difficulty of supplying raw materials for ethylene, where 2-methylalkanes can be used as ethylene raw materials, whilst due to the influence by the molecular structure, the yield of ethylene and triene is low in the process of free radical reaction such as steam cracking, and thus 2-methylalkanes are not ideal raw materials for ethylene.

In the prior art, a mature process and a matched catalyst are provided for conversion of isobutane, so that the normalization reaction of isobutane can be realized. However, no research has been disclosed on such 2-methylalkanes having a relatively high carbon number. If these small molecular 2-methylalkanes can be converted into n-alkanes and used as ethylene raw materials, the yield of ethylene product can be greatly increased, resulting great economic benefits.

### Summary of the Invention

The inventors of the invention find that small molecular iso-alkanes mainly undergoes two reaction processes of cracking and normalization in the hydroconversion process, wherein the conversion by the cracking reaction results in more low-carbon alkanes, while the conversion of iso-alkanes by the normalization reaction results in iso-alkanes with the same carbon number. By adjusting the catalyst and the process conditions, the molecular composition of the hydrogenation product can be flexibly adjusted, and the production flexibility is improved.

The inventors of the present invention also find that for 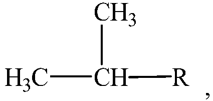 a paraffinic alkane with specific structure which is present in a large amount in petroleum processing, when R =1, compared with the case where R > 1, the energy consumed for its conversion is much higher, the reaction conditions are also more severe, and it is difficult to convert it together with the feedstock; while when R>6, the molecule is relatively large, which is thus very easy to be converted in a conventional hydrocracking process, with low processing difficulty. When R is 2-6, the molecule is relatively small, and the reaction is very difficult for either dehydrogenation or cracking of alkanes, so that it is difficult to convert it by a conventional processing means. Aiming at the defects of the prior art, the invention provides a process for converting iso-alkanes with the specific molecular structure, which can effectively convert the iso-alkanes into C₂-C₆ n-alkanes, and further can greatly improve the target product yield of an ethylene device when the iso-alkane is used as an ethylene raw material.

The present invention aims to produce C₂-C₆ n-alkanes in a maximized amount. In the prior art, C₅-C₆ iso-alkanes are usually converted into n-alkanes where only normalization reaction occurs, while cracking reaction is inhibited, wherein the whole normalization reaction process needs reduced reaction temperature and reduced hydrogen-to-oil volume ratio. However, the normalization reaction is influenced by chemical equilibrium, such that when the concentration of n-alkanes in the product reaches a certain proportion (about 30%), the normalization reaction is inhibited, and it is difficult to improve the yield of normal hydrocarbons in the product by adjusting the reaction process. Hence, the reaction route needs to be combined with separation of normal hydrocarbons and isomerized hydrocarbons, so as to separate out n-alkanes followed by further conversion of the unconverted iso-alkanes. However, C₂-C₆ n-alkanes are all ideal raw materials for preparing ethylene by steam cracking, for which the invention uses a special catalyst and controls the reaction conditions, so as to strengthen the both occurrences of normalization and cracking reaction at the same time, which breaks the influence of chemical equilibrium on the normalization reaction, wherein the yield of n-alkanes in the converted product are further improved by cracking reaction, which is particularly suitable for being directly used as a raw material for steam cracking to prepare ethylene. The present invention has been developed based on this object.

Specifically, the present invention relates to the following aspects.
1. A process for chemical conversion of a hydrocarbon stream, comprising the steps of:
   1) providing a hydrocarbon stream comprising a paraffinic alkane represented by the structural formula (I) of: 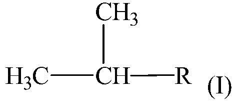 in which formula (I), R is a C₂-C₆ linear or branched alkyl (preferably a C₂-C₄ linear or branched alkyl, more preferably a C₂-C₃ linear alkyl),
   2) converting at least a part (such as 30wt% or more, 40wt% or more, 50wt% or more, 60wt% or more, 70wt% or more, 80wt% or more or 90wt% or more of the total amount) of the paraffinic alkanes in a conversion reaction to C₂-C₆ n-alkanes in the presence of a catalyst and hydrogen gas to obtain a converted product, wherein the catalyst comprises a molecular sieve and a non-noble metal hydrogenation-active metal component,
   3) steam cracking the converted product to obtain a cracked product comprising ethylene.
2. The process according to any one of the preceding or subsequent aspects, wherein the molecular sieve is one or more selected from the group consisting of mordenite, ZSM molecular sieve, SAPO molecular sieve and EU-1 molecular sieve, more preferably one or more selected from the group consisting of mordenite and ZSM molecular sieve, especially preferably one or more selected from the group consisting of mordenite, ZSM-5 molecular sieve, ZSM-11 molecular sieve, ZSM-12 molecular sieve, ZSM-22 molecular sieve, ZSM-23 molecular sieve, ZSM-35 molecular sieve, Beta molecular sieve and ZSM-38 molecular sieve, especially one or more selected from the group consisting of mordenite and ZSM-5 molecular sieve.
3. The process according to any one of the preceding or subsequent aspects, wherein the catalyst further comprises a binder, typically alumina or silica (preferably alumina), and the binder is present in an amount of 5-65 wt% (preferably 10-35 wt%) based on the weight of the catalyst.
4. The process according to any one of the preceding or subsequent aspects, wherein the non-noble metal hydrogenation active metal component is one or more selected from the group consisting of a non-noble metal of Group VIB, a non-noble metal of Group VIII, an oxide thereof or a sulfide thereof, more preferably one or more selected from the group consisting of molybdenum, tungsten, cobalt, nickel, a sulfide thereof or an oxide thereof
5. The process according to any one of the preceding or subsequent aspects, wherein the non-noble metal of Group VIB (calculated as oxide) is present in an amount of 5.0-30.0 wt% (preferably 10-20 wt%) and the non-noble metal of Group VIII (calculated as oxide) is present in an amount of 0.5-15.0 wt% (preferably 3 to 10 wt%), based on the weight of the catalyst.
6. The process according to any one of the preceding or subsequent aspects, wherein the molecular sieve is present in an amount of 30-80 wt% (preferably 40-70 wt%), based on the weight of the catalyst.
7. The process according to any one of the preceding or subsequent aspects, wherein the catalyst has a specific surface area of 200-400 m²/g and a pore volume of 0.25-0.45 mL/g.
8. The process according to any one of the preceding or subsequent aspects, wherein the conversion reaction is carried out under the reaction conditions of: a reaction pressure of 0.5-10.0 MPaG (preferably 2.0-8.0 MPaG or 2.0-5.0 MPaG), a reaction temperature of 300-500 °C (preferably 350-450 °C), a liquid hourly space velocity of 0.1-15.0 h⁻¹ (preferably 0.5-5.0 h⁻¹), and a volume ratio of hydrogen to oil of 50:1-2500:1 (preferably 100:1-2000:1 or 100:1-1000:1).
9. The process according to any one of the preceding or subsequent aspects, wherein the steam cracking is carried out under the conditions of: a reaction temperature of 750-900 °C, a reaction pressure of 0.1-0.5MPaG, and a mass ratio of water to oil of 0.2-0.6.
10. The process according to any one of the preceding or subsequent aspects, wherein the hydrocarbon stream has an initial boiling point of 10 °C to 30 °C (preferably 15 °C to 25 °C) and an end boiling point of 50 °C to 100 °C (preferably 55 °C to 70 °C).
11. The process according to any one of the preceding or subsequent aspects, wherein in the hydrocarbon stream, the C₇+ hydrocarbons are present in an amount of 0-10wt% (preferably 0.5-5 wt%), the C5-6 iso-alkanes are present in an amount of 50 wt% or more (preferably 60-90wt%, more preferably 70-80 wt%), the C₅ to C₆ n-alkanes are present in an amount of 10-30wt% (preferably 15-25 wt%), the C₄-hydrocarbons are present in an amount of 0-10wt% (preferably 2-5 wt%), and the cyclic hydrocarbons are present in an amount of 1 to 10wt% (preferably 2-5 wt%), based on the total weight of the hydrocarbon stream being 100 wt%.
12. The process according to any one of the preceding or subsequent aspects, wherein the converted product comprises C₂-C₃ alkanes and C₄-C₆ n-alkanes (preferably C₅-C₆ n-alkanes), and the weight ratio of the C₂-C₃ alkanes to the C₄-C₆ n-alkanes (preferably C₅-C₆ n-alkanes) is 0.5:1-8:1 (preferably 0.9:1-5:1).
13. The process according to any one of the preceding or subsequent aspects, wherein in the converted product, C₇⁺ hydrocarbons are present in an amount of not more than 5wt% (preferably not more than 1 wt%), C₅-C₆ iso-alkanes are present in an amount of 0-50 wt% (preferably 10-40 wt%), C₂-C₆ n-alkanes are present in an amount of 40-90wt% (preferably 50-80 wt%), cyclic hydrocarbons are present in an amount of not more than 3wt% (preferably not more than 1 wt%), based on the total weight of the converted product being 100 wt%.
14. The process according to any one of the preceding or subsequent aspects, wherein the hydrocarbon stream is a light naphtha component obtained by cracking (such as hydrocracking or catalytic cracking) a hydrocarbon oil feedstock (.
15. The process according to any one of the preceding or subsequent aspects, wherein the step 3) comprises the steps of:
   3-A-1) separating the converted product (called as a first separation) to obtain a separated stream with C₂+ hydrocarbons as the main part (such as representing 95 wt% or more, 98 wt% or more, 99 wt% or more, or essentially 100 wt% of the total amount),
   3-A-2) steam cracking the separated stream to obtain a cracked product comprising ethylene.
16. The process according to any one of the preceding or subsequent aspects, wherein the first separation is operated under conditions comprising a pressure of 0.5-10.0 MPaG (preferably 2.0-8.0 MPaG or 2.0-5.0 MPaG) and a temperature of 40-70 °C.
17. The process according to any one of the preceding or subsequent aspects, wherein the step 3) comprises the steps of:
   3-B-1) separating the converted product (called as a first separation) to obtain a separated stream with C₂+ hydrocarbons as the main part (such as representing 95 wt% or more, 98 wt% or more, 99 wt% or more, or essentially 100 wt% of the total amount),
   3-B-2) separating the separated stream (called as a second separation) to obtain a high-carbon stream comprising C₄+ hydrocarbons as the main part (such as 90 wt% or more, 95 wt% or more, 98 wt% or more, 99 wt% or more, or substantially 100 wt% of the total amount) and a low-carbon stream comprising C₂-C₃ hydrocarbons as the main part (such as 90 wt% or more, 95 wt% or more, 98 wt% or more, 99 wt% or more, or substantially 100 wt% of the total amount),
   3-B-3) optionally recycling the high-carbon stream as a hydrocarbon stream back to step 2) for the conversion reaction,
   3-B-4) steam cracking the low-carbon stream to obtain a cracked product comprising ethylene.
18. The process according to any one of the preceding or subsequent aspects, wherein the first separation is operated under conditions comprising a pressure of 0.5-10.0 MPaG (preferably 2.0-8.0 MPaG or 2.0-5.0 MPaG) and a temperature of 40-70 °C, and the second separation is operated under conditions comprising a pressure of 0.5-1.5 MPaG and a temperature of 30-60 °C.
19. A chemical conversion system comprising the units of:
   1) a hydrocarbon stream providing unit configured to be capable of providing a hydrocarbon stream comprising a paraffinic alkane represented by formula (I) of, 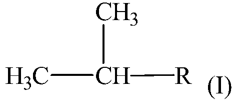 in the structural formula (I), R being a C2-6 linear or branched alkyl (preferably a C2-4 linear or branched alkyl, more preferably a C2-3 linear alkyl),
   2) a hydrocarbon stream conversion unit configured to convert at least a part (such as 30 wt% or more, 40 wt% or more, 50 wt% or more, 60 wt% or more, 70 wt% or more, 80 wt% or more, or 90 wt% or more of the total amount) of the paraffinic alkanes to C₂-C₆ n-alkanes in the presence of a catalyst and hydrogen, to obtain a converted product, wherein the catalyst comprises a molecular sieve and a non-noble metal hydrogenation active metal component,
   3) a steam cracking unit configured to be capable of steam cracking the converted product, to obtain a cracked product comprising ethylene.
20. A hydrocarbon mixture comprising C₅-C₆ n-alkanes and C₂-C₃ alkanes, wherein the C₅-C₆ n-alkanes are present in an amount of 5-30 wt% (preferably 10-25 wt%) and the C₂-C₃ alkanes are present in an amount of 20-50 wt% (preferably 25-45 wt%), based on 100wt% of the total weight of the hydrocarbon mixture.

### Technical effects

Compared with the prior art, the process and the system provided by the invention can achieve one of the following technical effects or the combination of all or part of the following technical effects:
1. Small molecular iso-alkanes, especially iso-alkanes having a methyl branch at the second carbon atom of a straight chain paraffinic alkane, are present in a large amount in both crude oil and petroleum-derived products, which amount is much higher than that of iso-alkanes of other molecular structures. The iso-alkanes with such a specific molecular structure has great problems when being used as either a product or in subsequent processing, and thus is not a good-quality product or raw material. The inventors of the invention find that the iso-alkanes with such a specific molecular structure can be effectively converted into n-alkanes by selecting a molecular sieve with a special pore structure, loaded with a certain amount of hydrogenation metal, which ensure the catalyst having stronger dehydrogenation performance to effectively convert the iso-alkanes to promote enhanced conversion to n-alkanes, and can greatly improve the target product yield of an ethylene device when used as an ethylene raw material.
2. The conversion process of the invention is simple, and the overall investment is low for either existing hydrogenation devices (including naphtha hydrogenation, aviation kerosene, diesel oil hydrogenation and wax oil hydrogenation) to be simply modified or new devices to be built; and the process, matched with a hydro-conversion catalyst with lower manufacturing cost, can efficiently convert the iso-alkanes into n-alkanes, with the advantages of low investment and high benefit.
3. When C₅-C₆ iso-alkanes are directly used as raw material for ethylene, the yield of either ethylene or triene is lower, while the ratio of C₂-C₃ low-carbon alkanes to C₄-C₆ n-alkanes in the hydrogenation product can be flexibly adjusted through hydrogenation conversion. Depending on the two different reaction routes of C₅-C₆ iso-alkanes, the two different reaction routes of cracking and normalization can be flexibly adjusted by adjusting the acid strength of the acid center of the catalyst. If the hydrogen is of lower cost or the hydrogen is of higher surplus, then more C₂-C₃ low-carbon alkanes can be produced, so that the yield of the target product of ethylene is improved greatly; whilst if the hydrogen is of higher cost or the hydrogen is of lower surplus, more C₄-C₆ n-alkanes can be produced, and the improvement of the ethylene raw material quality is realized with lower hydrogen consumption.
4. The hydrocarbon structure composition of the ethylene raw material is flexibly adjusted, and the operation flexibility of the ethylene device can be realized to the maximum extent, including flexibly adjusting the yield of main target products of ethylene, propylene, butadiene and aromatic hydrocarbons of the ethylene device, such that the product structure can be adjusted according to the change of market demands, and the market competitiveness of the ethylene device is improved.
5. The hydro-converted product has high content of C₂-C₆ n-alkanes, especially C₂-C₃ small molecular n-alkanes, such that better ethylene yield can be obtained in the steam cracking process, and meanwhile, C₄-C₆ n-alkanes in the hydro-converted product is greatly improved compared with the raw material, which thus can be directly used as an ethylene raw material.
6. The present invention aims at maximizing the production of C₂-C₆ n-paraffinic alkanes, while the prior art usually converts C₅-C₆ iso-alkanes into n-alkanes, i.e., only normalization reaction occurring while inhibiting cracking reactions, where the whole normalization reaction process needs to be carried out at a reduced reaction temperature and a reduced hydrogen-to-oil volume ratio. However, the normalization reaction is influenced by chemical equilibrium, and the normalization reaction is inhibited after the n-alkane concentration in the product reaches a certain ratio (about 30%), such that the yield of n-alkanes in the product is difficult to be improved by adjusting the reaction process, and the process needs to be combined with separation of n- and iso-alkanes to separate out n-alkanes and then to convert the unconverted iso-alkanes. However, C₂-C₆ n-alkanes are all ideal raw materials for preparing ethylene by steam cracking, and the invention uses a special catalyst and controls the reaction conditions, strengthens the occurrences of both normalization and cracking reaction at the same time, breaks the influence of chemical equilibrium on the normalization reaction, and further improves the yield of n-alkanes in the converted product by the cracking reaction, where the latter is particularly suitable for being directly used as a steam cracking raw material to prepare ethylene. The present invention has been developed based on this object.

### Description of Drawings

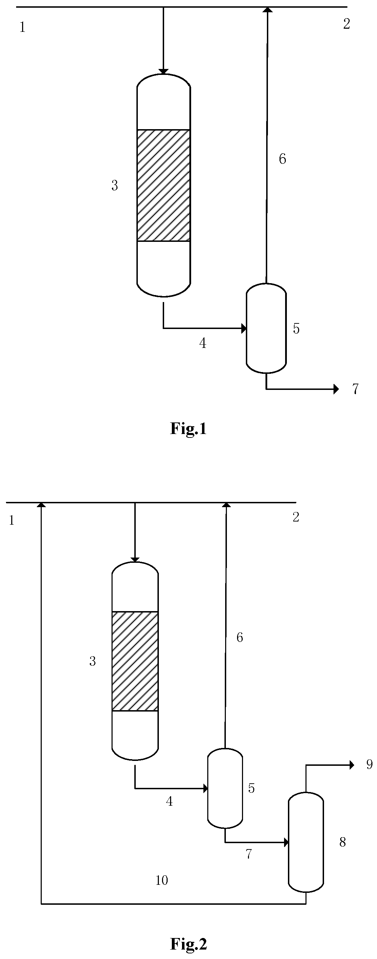

Fig.1 is a schematic figure of the process according to the present invention.
   In Fig.1, 1-hydrocarbon stream, 2-hydrogen, 3-hydroconversion reaction zone, 4-hydroconversion reaction zone effluent, 5-high pressure separator, 6-hydrogen-enriched gas-phase stream, 7-liquid-phase stream.
Fig.2 is a schematic figure of the process according to the present invention.
   In Fig.2, 1-hydrocarbon stream, 2-hydrogen, 3-hydroconversion reaction zone, 4-hydroconversion reaction zone effluent, 5-high pressure separator, 6-hydrogen-enriched gas-phase stream, 7-liquid-phase stream, 8-fractionation column, 9-gas-phase product, 10-liquid-phase product.

### Embodiments of the Invention

The embodiments of the present invention will be illustrated in more detail below, but it should be understood that the scope of the invention is not limited by the embodiments, but is defined by the claims appended.

All publications, patent applications, patents, and other references mentioned in this specification are herein incorporated by reference in their entirety. Unless defined otherwise, all technical and scientific terms used herein are understood same as the meanings commonly known to those skilled in the art. In case of conflict, definitions according to the present specification will control. When the specification introduces materials, substances, processes, steps, devices, components, or the like initiated with "known to those ordinary skill in the art", "prior art", or the like, it is intended that the subject matter so initiated encompass not only those conventionally used in the art at the time of filing this application, but also those may not be so commonly used at the present time, but will become known in the art as being suitable for a similar purpose. In the context of the present invention, all numerical values of a parameter (e.g., quantity or condition) are to be understood as being modified in all instances by the term "about", whether or not "about" actually appears before the numerical value.

In the context of the present invention, each device used in the present invention may adopt a structure conventionally selected in the art without being particularly limited thereto.

In the context of this specification, the term "substantially" means a deviation acceptable or considered as reasonable by a person skilled in the art, such as within ± 5%, within ±2%, within ± 1%, within ±0.5% or within ±0.1%. In the context of the present invention, the initial boiling point and the end boiling point are measured according to the method of ASTM D86.

In the context of the present invention, the term " C₂-C₆ n-alkanes" refers to n-alkanes having from 2 to 6 carbon atoms. It is to be specifically understood that although the alkanes having from 2 to 3 carbon atoms cannot be classified into n-alkane and iso-alkane, the alkanes having from 2 to 3 carbon atoms are also called as n-alkanes in the present invention for convenience of description. In addition, when the term is used in the present invention, the term may refer to any one or more of n-alkanes having from 2 to 6 carbon atoms, or all of n-alkanes having from 2 to 6 carbon atoms, unless otherwise specified.

In the context of the present invention, a cyclic hydrocarbon refers to a hydrocarbon having a cyclic structure in the molecular structure, such as cycloalkanes and aromatics.

All percentages, parts, ratios, and the like referred to in the specification are based on weight, and pressures denote gauge pressures unless explicitly indicated.

In the context of the present invention, any two or more embodiments or aspects of the present invention may be arbitrarily combined, and the technical solutions resulted are part of the original disclosure of the present specification, and also fall within the scope of the present invention.

According to an embodiment of the invention, it relates to a chemical conversion process, in particular a process for chemical conversion of a hydrocarbon stream or paraffinic alkanes, which allows for flexible adjustment of the converted product composition. According to the invention, the molecular sieve type and the acid center strength of the catalyst, and the process parameters can be adjusted to provide a converted product having a weight ratio of C₂-C₃ hydrocarbons to C₄-C₆ n-alkanes flexibly adjusted within the range of 0.5:1-8:1 (preferably 0.9:1-5:1), so that different hydrocarbon components can be obtained useful for ethylene raw materials. A converted product with higher content of C₂-C₃ hydrocarbons has higher content of normal hydrocarbons, but the conversion process thereof consumes more hydrogen; while a converted product with higher content of C₄-C₆ n-alkanes content consumes less hydrogen during the reaction process, which also improves the quality of the ethylene raw material. Here, the C₄-C₆ n-alkanes are more preferably C₅-C₆ n-alkanes in view of more excellent achievement of the technical effect of the present invention.

According to an embodiment of the invention, the chemical conversion process comprises the steps of: 1): providing a hydrocarbon stream comprising a paraffinic alkane represented by the following structural formula (I). Here, the paraffinic alkane is represented by the following structural formula (I).

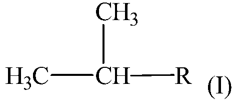

According to an embodiment of the invention, in formula (I), R is a C₂-C₆ linear or branched alkyl, preferably C₂-C₄ linear or branched alkyl, more preferably C₂-C₃ linear alkyl. When R is a C₂-C₃ linear alkyl, the paraffinic alkane is sometimes called briefly as a C₅-C₆ iso-alkane. The inventors of the present invention find that when R =1, the conversion needs much higher energy consumption and more severe reaction conditions compared with when R > 1, and it is difficult to convert it together with other feedstock. When R>6, the molecular structure of the alkane is large, which is thus very easy to be converted in a conventional hydrocracking process, with low processing difficulty. The inventors of the present invention also find that when R has a branched chain, the reaction of two tertiary carbon atoms to be dehydrogenated simultaneously to generate a tertiary carbonium ion will be much more difficult, where the iso-alkane with such a molecular structure does not undergo a normalization reaction, but only one reaction route, namely a cracking reaction, is available, so that the hydrogen consumption is greatly increased; meanwhile, the branched chain structure is complex, being difficult to enter the molecular sieve pore channel, resulting in being difficult to be cracked, and at least one iso-alkane is still produced in the converted product after the cracking reaction due to the existence of two methyl branched chains, so the conversion effect is poor. For this reason, in order to improve the technical effect of the present invention, the content of iso-alkanes other than the paraffinic alkanes of the structural formula (I), such as C₅-C₆ iso-alkanes of other isomer structures, in the hydrocarbon stream is controlled as low as possible, such as generally 20 wt% or less or 10 wt% or less, based on 100wt% of the total weight of the hydrocarbon stream.

According to the present invention, the paraffinic alkane or hydrocarbon stream may be produced by any process known in the art. Here, as a process for producing the paraffinic alkane or the hydrocarbon stream, for example, hydrocracking, catalytic cracking, atmospheric vacuum distillation, or the like may be mentioned, so as to produce the paraffinic alkane or the hydrocarbon stream. Preferably, the hydrocarbon stream has an initial boiling point in the range of 10-30 °C (preferably 15-25 °C) and an end boiling point in the range of 50-100 °C (preferably 55-70 °C). More preferably, the hydrocarbon stream is a light naphtha component obtained by subjecting a hydrocarbon oil feedstock to hydrocracking. In addition, the hydrocarbon stream may be used in the conversion reaction described hereinafter in the present invention with or without separation (such as rectification or adsorption), but preferably without separation from the viewpoint of the cost of obtaining the raw material.

According to an embodiment of the present invention, in order to achieve the technical effects of the present invention, the content of the C₅-C₆ iso-alkanes or the paraffinic alkanes in the hydrocarbon stream is generally 50 wt% or more (preferably 60-90wt%, more preferably 70-80 wt%), based on the total weight of the hydrocarbon stream being 100 wt%. According to the present invention, the conversion of the iso-alkanes in the hydrocarbon stream is influenced not only by the type of catalyst and the process conditions, but also by the molecular composition of the feedstock, i.e. a higher amount of iso-alkanes in the feedstock. If the content of iso-alkanes in the feedstock is low, the content of n-alkanes in the converted product is limitedly increased relative to the content of n-alkanes in the feedstock, such that the output of target product is limitedly increased when the product is used as an ethylene raw material. If the content of iso-alkane in the feedstock is high, then the n-alkanes content in the converted product can be greatly increased after conversion, whilst the feedstock with high content of iso-alkanes is difficult to be obtained, which is difficult to be directly obtained by a conventional separation process, so that the cost of obtaining the hydrocarbon stream is remarkably increased.

According to an embodiment of the present invention, the content of C₇₊ hydrocarbons in the hydrocarbon stream is not particularly limited, but for a better technical effect of the present invention, the content of C₇₊ hydrocarbons in the hydrocarbon stream is generally 0-10wt% (preferably 0.5-5 wt%), based on the total weight of the hydrocarbon stream being 100 wt%. The inventors of the present invention find that, for the hydroconversion catalyst of the present invention, because the molecular sieve has a relatively special pore structure, the macromolecular iso-alkanes and cyclic hydrocarbons in the reactant are difficult to enter the pores of the catalyst for conversion (for example, the conversion rate is generally lower than 10%), therefore, a too high content of C₇₊ hydrocarbons may affect the conversion effect of the hydrocarbon stream of the present invention, and is not favorable for the present invention.

According to an embodiment of the present invention, the content of C₅-C₆ n-alkanes in the hydrocarbon stream is not particularly limited, but for a better technical effect of the present invention, the content of C₅-C₆ n-alkanes in the hydrocarbon stream is generally 10-30wt% (preferably 15-25 wt%), based on the total weight of the hydrocarbon stream being 100 wt%. The inventors of the present invention find that, due to the presence of two reaction routes of normalization and cracking reaction in the hydroconversion process, the content of n-alkanes in the hydrocarbon stream has little influence on the cracking reaction, but can influence the normalization reaction, where the normalization reaction can be inhibited by excessively high content of normal hydrocarbons. Meanwhile, when a raw material with higher content of normal hydrocarbons is directly used as an ethylene raw material, higher yield of ethylene product can be obtained, while the quality of the hydrocarbon stream after hydroconversion may only be limitedly improved.

According to an embodiment of the present invention, the content of C₄hydrocarbons in the hydrocarbon stream is not particularly limited, but for a better technical effect of the present invention, the content of C₄- hydrocarbons in the hydrocarbon stream is generally 0-10wt% (preferably 2-5 wt%), based on the total weight of the hydrocarbon stream being 100 wt%. The inventors of the present invention find that C₄- hydrocarbons have significantly different physical properties, especially boiling points, from C₅-C₆ hydrocarbons, which also result in mixing of the two streams more difficult. In addition, the inventors of the present invention find that conversion of C₄- hydrocarbons consumes much higher energy than that of C₅-C₆ iso-alkanes, and the reaction performance is poor when a C₄ hydrocarbon and C₅-C₆ iso-alkanes are mixed (for example, the conversion rate is generally lower than 20%), so that a too high content of C₄ hydrocarbon affects the conversion effect of C₅-C₆ iso-alkane, which is not favorable for the present invention.

According to an embodiment of the present invention, the content of cyclic hydrocarbons in the hydrocarbon stream is not particularly limited, but for a better technical effect of the present invention, the content of cyclic hydrocarbons in the hydrocarbon stream is generally in the range of 1-10 wt% (preferably 2-5 wt%), based on the total weight of the hydrocarbon stream being 100 wt%. The inventors of the present invention find that, as the number of carbons of a cyclic hydrocarbon increases, the molecular diameter of the cyclic hydrocarbon gradually increases, resulting in the macromolecular cyclic hydrocarbon difficult to enter the catalyst pore channels for reaction, while the cyclic hydrocarbons not subjected to the hydrogenation conversion entered the ethylene device will affect the yield of the target product of the ethylene device, which is disadvantageous in the present invention.

According to an embodiment of the invention, the chemical conversion process comprises step 2): converting at least a part of the C₅-C₆ iso-alkanes or the paraffinic alkanes into C₂-C₆ n-alkanes in the presence of a catalyst and hydrogen, to obtain a converted product. Here, the said "at least a part" of a total amount is, for example, 30wt% or more, 40wt% or more, 50wt% or more, 60wt% or more, 70wt% or more, 80wt% or more, or 90wt% or more. According to the present invention, the conversion rate of the C₅-C₆ iso-alkanes or the paraffinic alkanes is not particularly limited, and therefore, the paraffinic alkanes or the C₅-C₆ iso-alkanes may be converted once (one-pass conversion rate) to achieve the conversion rate desired, or may be recycled for several times (for example, after separation of unreacted paraffinic alkanes or iso-alkanes from the converted product and subsequent conversion) to achieve the conversion rate. For example, the conversion rate (preferably one-pass conversion rate) may be 30wt% or more, 40wt% or more, 50wt% or more, 60wt% or more, 70wt% or more, 80wt% or more, or 90wt% or more.

According to an embodiment of the present invention, the conversion reaction may be a hydro-normalizing conversion reaction to obtain a converted product with C₄-C₆ n-alkanes as the main product, or a hydrocracking reaction to obtain a converted product with C₂-C₃ paraffinic alkane as the main product. The inventors of the present invention find that the paraffinic alkanes or the C₅-C₆ iso-alkanes need a molecular sieve with a special pore structure to be effectively converted, and meanwhile, the catalyst should have strong hydrogenation/dehydrogenation activity. The iso-alkanes with the special structure are firstly subjected dehydrogenation reaction on a hydrogenation center to generate olefin, and the molecular structure of the olefin molecule is then changed on an acid center transferred. If it is shifted to a strong acid center, a cracking reaction is easier to occur; whilst if it is shifted to a medium-strong acid center, a normalization reaction is easier to occur. The two reaction routes can be respectively strengthened by selecting molecular sieves with different reaction characteristics.

According to an embodiment of the invention, the reaction conditions for the conversion reaction include: a reaction pressure of 0.5-10.0MPaG (preferably 2.0-8.0MPaG or 2.0-5.0 MPaG), a reaction temperature of 250-500 °C, preferably 300-500 °C or 350-450 °C, a liquid hourly space velocity of 0.1-15.0 h⁻¹ (preferably 0.5-5.0 h⁻¹), and a hydrogen-oil volume ratio of 10:1-2500:1, preferably 50:1-2500:1, 100:1-2000:1 or 100:1-1000:1. During the process of hydroconversion of C₅-C₆ iso-alkanes, both normalization reaction and cracking reaction are typically endothermic reactions, where the higher the reaction temperature is, the more favorable the chemical reaction is to proceed towards the positive reaction direction. A too low reaction temperature can inhibit both the normalization reaction and the cracking reaction, thus seriously affecting the iso-alkane conversion. C₅-C₆ iso-alkanes are in gas form at a high temperature, such that during the gas-phase reaction, the hydrogen-oil ratio is represented as the hydrogen concentration per unit volume, and a higher hydrogen concentration is beneficial for hydrogen to be bound with reactants, so as to promote the normalization and hydrogenolysis reaction.

The inventors of the present invention find that the paraffinic alkanes or the C₅-C₆ iso-alkanes may exhibit a one-pass conversion rate of 40wt% or more, 50wt% or more, 60wt% or more, 70wt% or more, 80wt% or more, or 90wt% or more under the reaction conditions of the present invention. The inventors of the present invention further find that the conversion reaction is specific for the paraffinic alkanes or the C₅-C₆ iso-alkanes.In the conversion of a paraffinic alkane having a relatively high carbon number (for example, R =7 or more), the normalization becomes more difficult to occur while the cracking reaction becomes easier to occur, with the increase of the number of carbon atoms. For the conversion of a component having R =7 or more, normalization reaction hardly occurs during the reaction, while an excessive cracking reaction greatly increases the consumption of hydrogen, resulting in poor conversion effect. In addition, as to the conversion of C₇₊ components, for the hydroconversion catalyst of the present invention, due to the relatively special pore structure of the molecular sieve, the macromolecular iso-alkanes and cyclic hydrocarbons in the reactant are difficult to enter the catalyst pore for conversion (for example, the conversion rate is generally lower than 10%), therefore, a too high content of C₇₊ hydrocarbons may affect the conversion effect of the hydrocarbon stream of the present invention. The inventors of the invention find that in the process of the hydroconversion of low-carbon hydrocarbons, the reactant is firstly subjected to a dehydrogenation reaction to generate carbonium ions, and the bond energy between C-C bonds is gradually increased along with the reduction of the molecular weight of the hydrocarbon, namely, the smaller the molecular weight is, the more difficult the dehydrogenation reaction occurs, so that C₄ is difficult to be reacted under conversion conditions appropriate for C₅-C₆ iso-alkanes, and harsher reaction conditions are required for sufficient reaction of a C₄ component, which causes excessive cracking of C₅-C₆ iso-alkanes, resulting in great increase of hydrogen consumption of the reaction, which reduces the economic benefit.

According to the invention, the catalyst comprises a molecular sieve and a non-noble metal hydrogenation active metal component.According to the invention, the molecular sieve is one or more selected from the group consisting of mordenite, ZSM molecular sieve, SAPO molecular sieve and EU-1 molecular sieve, more preferably one or more selected from the group consisting of mordenite and ZSM molecular sieve, particularly preferably one or more selected from the group consisting of mordenite, ZSM-5 molecular sieve, ZSM-11 molecular sieve, ZSM-12 molecular sieve, ZSM-22 molecular sieve, ZSM-23 molecular sieve, ZSM-35 molecular sieve, Beta molecular sieve and ZSM-38 molecular sieve, and especially one or more selected from the group consisting of mordenite and ZSM-5 molecular sieve. Here, the molecular sieve is present in an amount of 30-80wt% (preferably 40-70 wt%), based on the weight of the catalyst. The inventors of the invention find that the acid strength of the molecular sieve directly influences the reaction behaviors of iso-alkane, where the molecular sieves having more strong acid centers are more likely to cause a cracking reaction, such as ZSM-5 molecular sieve; while the molecular sieves having more medium-strong acid centers are more likely to cause a normalization reaction, such as mordenite. Thus, according to the present invention, different reaction routes can be achieved by selection of molecular sieve acid strength.

The inventors of the invention find that in the conversion process of C₅-C₆ iso-alkanes, the pore structure of the molecular sieve directly affects the reaction effect. For example, a molecular sieve such as Y molecular sieve having a super cage structure has large pore channels, such that the retention time of small molecular iso-alkanes in the pore channels of the catalyst is short, resulting in the reaction difficult to be effectively carried out. Meanwhile, when a cracking reaction occurs, the large pore structure can cause excessive amount of isobutane generated, which is not beneficial to improve the yield of n-alkanes in the converted product.

The inventors of the invention find that two types of reaction routes are mainly available in the hydroconversion process, one being a cracking reaction for converting to C₂-C₃ hydrocarbons, and the other being a normalization reaction for generating C₄-C₆ n-alkanes. Generally, the two types of reactions occur simultaneously, but different reaction processes can be respectively enhanced by adjusting the molecular sieve and the acid strength of the acid center. A strong acid center is favorable for the cracking reaction, while a medium-strong acid center is favorable for the normalization reaction. For example, a mordenite containing more medium-strong acid results in more normalization reactions; while a ZSM-5 molecular sieve containing more strong acid results in more cracking reactions. According to the invention, the weight ratio of C₂-C₃ hydrocarbons to C₄-C₆ n-alkanes in the converted product can be flexibly adjusted by adjusting the weight ratio of mordenite to ZSM-5 molecular sieve in the range of 0-100:100-0 (preferably 10-50:50-10).

The inventors of the invention find that the chemical conversion reaction may be a hydro-normalization reaction to obtain a converted product with C4-C6 n-alkanes as the main product, or may also be a hydrocracking reaction to obtain a converted product with C2-C3 hydrocarbons as the main product. The inventors of the invention find that C5 and C6 iso-alkanes mainly involve two reaction routes in the hydroconversion process, wherein one reaction route is a cracking reaction for converting to generate C2-C3 hydrocarbons, and the other reaction route is a normalization reaction for generating C4-C6 n-alkanes. In general, the two reactions occur simultaneously, but different reaction processes can be respectively enhanced by adjusting the molecular sieve and the acid strength of the acid center. A strong acid center is favorable for the cracking reaction, while a medium-strong acid center is favorable for the normalization reaction. Through such reaction principle, the generation ratio of the C2-C3 hydrocarbons to the C4-C6 n-alkanes in the converted product can be flexibly adjusted. The inventors of the present invention find that the content of iso-alkanes in the raw material also has a direct influence on the conversion process. For a cracking reaction, although the reactant is not limited to be n-alkanes or iso-alkanes, the higher the content of iso-alkanes in the raw material is, the higher the probability of cracking reaction is, and the quality of the converted product can be improved more. For a normalization reaction, the reactant only involves iso-alkanes, and the normalization reaction is influenced by chemical equilibrium, where a higher content of iso-alkanes is more beneficial to the conversion reaction towards normalization.

According to an embodiment of the present invention, the non-noble metal hydrogenation active metal component is one or more selected from the group consisting of a non-noble metal of Group VIB, a non-noble metal of Group VIII, an oxide thereof or a sulfide thereof, more preferably one or more selected from the group consisting of molybdenum, tungsten, cobalt, nickel, a sulfide thereof or an oxide thereof.Here, the non-noble metal of Group VIB (as oxide) is present in an amount of 5.0-30.0 wt% (10.0-20.0 wt%), and the non-noble metal of Group VIII (as oxide) is present in an amount of 0.5-15.0 wt% (preferably 3.0-10.0 wt%), based on the weight of the catalyst. Preferably, the active metal component of the present invention does not contain a noble metal such as Pt or the like. The inventors of the present invention find that a noble metal component has a stronger hydrogenation/dehydrogenation performance than a non-noble metal component, and can cause a dehydrogenation reaction at a lower reaction temperature to convert C₅-C₆ iso-alkanes into C₅-C₆ n-alkanes, while it is known that a cracking reaction is mainly affected by the reaction temperature, and the lower reaction temperature and the stronger hydrogenation performance suppress the cracking reaction. Therefore, use of a noble metal catalyst, result in difficult to produce more C₂-C₃ alkanes in an operation aiming at normalization. As compared, the non-noble metal catalyst has relatively weak dehydrogenation performance, so that the reaction needs a higher reaction temperature, and a large scale of cracking reaction is accompanied with the normalization reaction, so that more C₂-C₃ alkanes are generated.

According to an embodiment of the invention, the catalyst further comprises a binder. The binder may be of any conventional type in the art and comprises, but not limited to, alumina and silica, preferably alumina. Here, the binder is present in an amount of 5-65 wt% (preferably 10-35 wt%), based on the weight of the catalyst.

According to an embodiment of the invention, the catalyst has a specific surface area of 200-400 m²/g, and a pore volume of 0.25-0.45 mL/g.

According to an embodiment of the present invention, the catalyst may be prepared according to a conventional process in the art. The preparation process comprises the steps of preparing the support and loading the active metal component, wherein the support is prepared by: mechanically mixing the molecular sieve and the binder, molding, drying and calcinating, to prepare the catalyst support. The drying and calcination of the support may be carried out under conventional conditions. Conditions for drying may comprise: drying for 1-12 hours at 100-150 °C. Conditions for calcination may comprise: calcinating at 450-550 °C for 2.5-6.0 hr.

According to an embodiment of the present invention, for the preparation process of the catalyst, the active metal component is loaded by a conventional process such as a kneading process, an impregnation process, and the like, preferably an impregnation process. The impregnation process can be a saturated impregnation process, an excess impregnation process or a complexing impregnation process, where the catalyst support is impregnated by a solution containing the active metal component, followed by drying and calcinating, to obtain said catalyst. Conditions for drying may comprise: drying at 100-150 °C for 1-12 hr. Conditions for calcination may comprise: calcinating at 450-550 °C for 2.5-6.0 hr.

According to an embodiment of the invention, the converted product comprises C₂-C₃ alkanes and C₄-C₆ n-alkanes (preferably C₅-C₆ n-alkanes).Preferably, the weight ratio of the C₂-C₃ alkanes to the C₄-C₆ n-alkanes (preferably C₅-C₆ n-alkanes) in the converted product is generally 0.5:1-8:1 (preferably 0.9:1-5:1).

According to an embodiment of the invention, the converted product has a content of C₇+ hydrocarbons of not more than 5wt% (preferably not more than 1 wt%), a content of cyclic hydrocarbons of not more than 3wt% (preferably not more than 1 wt%), based on the total weight of the converted product being 100 wt%. According to the present invention, the conversion reaction produces little amount of C₇+ hydrocarbons, and a very low amount of methane (e.g., less than 5 wt%). Methane is a low value-added product in petrochemical processes and can only be used as fuel gas; meanwhile, since the methane has the highest hydrogen content in all the hydrocarbons, excessive methane generated from a hydroconversion will result in inefficient loss of a large amount of hydrogen.

According to an embodiment of the invention, depending on the conversion rate, the converted product generally has an content of C₅ to C₆ iso-alkanes in the range of 0-50 wt% (preferably 10-40 wt%), based on the total weight of the converted product being 100 wt%. The hydroconversion process comprises two reaction routes of normalization and cracking, and the normalization is a more ideal reaction route because hydrogen is not consumed therein and the converted product do not contain ineffective components like iso-alkanes and methane. The normalization reaction is influenced by chemical equilibrium, namely the higher the content of iso-alkanes is, the more easy the normalization reaction is to occur, such that when the conversion rate is low, although more normalization reactions occur, the content of n-alkanes in the product is limitedly increased to a limited extent; while when the conversion rate is high, a large amount of iso-alkanes undergo cracking reaction, resulting in reduced concentration of iso-alkanes in the reactant, which also inhibits the occurrence of normalization reaction.

According to an embodiment of the invention, depending on the conversion rate, the converted product has a content of C₂-C₆ n-alkanes of 40-90 wt% (preferably 50-80 wt%), based on the total weight of the converted product being 100 wt%.

According to an embodiment of the present invention, the step 3) may comprise the steps of: separating the converted product (called as a first separation) to obtain a separated stream having C₂+ hydrocarbons as the main part (e.g., 95 wt% or more, 98 wt% or more, 99 wt% or more, or substantially 100 wt% of the total amount). Here, the C₂+ hydrocarbon means a hydrocarbon having 2 or more carbon atoms. To this end, according to the invention, the first separation is aimed at removing, by separation, hydrogen from the converted product, which hydrogen may have been formed during the conversion reaction or may be present as unreacted hydrogen, in order to avoid hydrogen adversely affecting the subsequent steam cracking step. According to the present invention, the first separation is not particularly limited, as long as it can effectively separate hydrogen from the converted product, and the first separation may be performed in any gas-liquid separation device conventionally known in the art, such as a flash drum, without being particularly limited. According to the invention, the first separation is generally operated under conditions comprising a pressure of 0.5-10.0MPaG (preferably 2.0-8.0MPaG or 2.0-5.0MPaG) and a temperature of 40-70 °C. Further according to the invention, the separated hydrogen can be recycled as a recycled stream to the step 2) for the conversion reaction.

According to an embodiment of the present invention, the step 3) may further comprise the steps of: separating the separated stream (called as a second separation) to obtain a high-carbon stream having C₄+ hydrocarbons as the main part (such as 90 wt% or more, 95 wt% or more, 98 wt% or more, 99 wt% or more, or substantially 100 wt% of the total amount) and a low-carbon stream having C₂-C₃ hydrocarbons as the main part (such as 90 wt% or more, 95 wt% or more, 98 wt% or more, 99 wt% or more, or substantially 100 wt% of the total amount). Here, the C₄+ hydrocarbon refers to a hydrocarbon having 4 or more carbon atoms, and encompasses C₄-C₆ iso-alkane and C₄-C₆ n-alkanes, while the C₂-C₃ hydrocarbon encompasses ethane and propane. To this end, according to the invention, the second separation is aimed at an efficient separation of hydrocarbons having 4 or more carbon atoms from hydrocarbons having 2 and 3 carbon atoms in the separated stream. However, the second separation is not intended to separate any isomers, e.g., the second separation being not intended to separate C₄-C₆ iso-alkanes from C₄-C₆ n-alkanes in the separated stream, which is a characteristic of the present invention. The invention aims to produce C₂-C₆ n-alkanes in maximum quantity. In the prior art, C₅-C₆ iso-alkanes are generally converted into n-alkanes, namely only normalization reaction occurs, while cracking reaction is inhibited, where the whole normalization reaction process prefers reduced reaction temperature and reduced hydrogen-oil volume ratio. However, the normalization reaction is influenced by chemical equilibrium, such that when the n-alkanes concentration in the product reaches a certain proportion (about 30%), the normalization reaction is inhibited, resulting in the yield of n-alkanes in the product difficult to be improved by adjusting the reaction process, which means this reaction route needs to be combined with a separation of n- and iso-alkanes, wherein after the n-alkanes are separated, the unconverted iso-alkanes are continuously converted. However, C₂-C₆ n-alkanes are ideal raw materials for preparing ethylene by steam cracking. The invention uses a special catalyst and controls reaction conditions, which strengthens the occurrence of both normalization and cracking reaction at the same time, breaking the influence of chemical equilibrium on the normalization reaction, so as to further improve the yield of n-alkanes in the converted product by conducting the cracking reaction, and thus is particularly suitable for being directly used as a steam cracking raw material to prepare ethylene. Generally, a steam cracking device comprises a plurality of reactors depending on different feeds, mainly because a reaction feed with lower molecular weight needs higher cracking temperature, such as a gas reactor and a liquid reactor. In the process of converting iso-alkanes, the distribution of the converted product can be flexibly adjusted according to different requirements. If the content of C₂-C₃ in the converted product is lower, a second part of separation is not needed, and the converted product is fed directly into a liquid reactor for conversion; while if the content of C₂-C₃ is higher, a second part of separation needs to be carried out, and the separated products are fed respectively into a gas reactor and a liquid reactor. The invention does not provide a traditional normalization reaction, namely, iso-alkanes being converted into n-alkanes with the same molecular weight, in which conversion process, in order to obtain higher yield of n-alkanes, a certain amount of cracking reaction is accompanied, where the generated C₂-C₃ alkanes are very easy to be separated from the liquid-phase product, and the liquid-phase product obtained after conversion is greatly reduced compared with the fresh feed, meanwhile, the proportion of n-alkanes is greatly improved, such that the separation of n- and iso-alkanes carried out subsequently will result in greatly increased energy consumption and reduced overall economic benefit.

According to the present invention, the second separation is not particularly limited, as long as it can effectively separate the C₄+ hydrocarbons from the C₂-C₃ hydrocarbons, and the second separation can be performed in any gas-liquid separation device conventionally known in the art, such as a rectifying column, without being particularly limited. According to the invention, the operating conditions of the second separation comprise a pressure of 0.5-1.5MPaG and a temperature of 30-60 °C. In the process of converting iso-alkanes, the distribution of the converted products can be flexibly adjusted according to different requirements, where if the content of C₂-C₃ in the converted product is low, the converted product does not need a second separation and can be directly fed into a liquid reactor for conversion, while if the content of C₂-C₃ is high, the converted product needs a second separation and the separated products are fed respectively into a gas reactor and a liquid reactor.

According to an embodiment of the present invention, the step 3) may further comprise the step of: recycling the high-carbon stream as a hydrocarbon stream back to the step 2) for the conversion reaction. The inventors of the present invention find that the high-carbon stream contains unreacted C₅-C₆ iso-alkanes and is therefore suitable for continuing the conversion reaction as a hydrocarbon stream.This recycling step present as an optional step, but preferably as an essential step.

According to an embodiment of the present invention, the step 3) may further comprise the step of: steam cracking the separated stream or the low-carbon stream, to obtain a cracked product comprising ethylene. The inventors of the present invention find that the low-carbon stream or the separated stream can be directly used as a feedstock for steam cracking without any additional treatment, such as separation to enrich the n-alkanes or separation to remove any light or heavy components. Here, the steam cracking may be performed in any manner conventionally known in the art, without being particularly limited. Generally, the operating conditions for the steam cracking comprise: a reaction temperature of 750-900 °C, a reaction pressure of 0.1-0.5MPaG, and a mass ratio of water to oil of 0.2-0.6.

According to an embodiment of the invention, the invention also relates to a hydrocarbon mixture comprising C₅-C₆ n-alkanes and C₂-C₃ alkanes. According to the invention, the hydrocarbon mixture can be obtained as the converted product by the chemical conversion process of the invention as described previously. According to the invention, the hydrocarbon mixture, as the converted product, consists essentially of C₂-C₇ alkanes, where the content of C₂-C₇ alkanes is generally 90wt% or more, based on the total weight of the hydrocarbon mixture being 100wt%.

According to an embodiment of the present invention, the C₅-C₆ n-alkanes are present in an amount of 5-30wt% (preferably 10-25 wt%), and the C₂-C₃ alkanes are present in an amount of 20-50wt% (preferably 25-45 wt%), based on the total weight of the hydrocarbon mixture being 100wt%. The inventors of the invention find that the normalization reaction of C₅-C₆ iso-alkanes is influenced by chemical equilibrium, such that when the n-alkanes reaches a certain concentration, the yield of normal hydrocarbons in the converted product is difficult to be improved through the normalization reaction, and the yield of normal hydrocarbons in the converted product needs to be improved by controlling a proper proportion of cracking reaction. Meanwhile, although excessive cracking reaction will cause the generation of a large amount of C₂-C₃ alkanes, it will also cause cracking reactions of a part of C₅-C₆ n-alkanes, so as to consume excessive hydrogen, resulting in limited improvement of the yield of n-alkanes in the converted product. The inventors of the present invention also find that C₅-C₆ n-alkanes can undergo more normalization reactions when mordenite is used as a molecular sieve having shape-selective catalytic properties, compared with the case of using a ZSM-5 molecular sieve, wherein the yield of C₅-C₆ n-alkanes in the hydrogenated product is higher, hydrogen is not consumed in the reaction process, the yield of propane in the hydrogenated product from the cracking reaction is higher, and the consumption of hydrogen is higher. Therefore, the hydroconversion process using mordenite molecular sieve consumes less hydrogen, and meanwhile, propane can generate a large amount of methane in the steam cracking reaction process when the hydrogenated product is used as an ethylene raw material, while C₅-C₆ n-alkanes result in a low yield of methane, thus having better economic benefits.

According to an embodiment of the invention, it also relates to a chemical conversion system comprising a hydrocarbon stream providing unit and a hydrocarbon stream conversion unit. According to the invention, the system is provided to conduct the process described above, such that in addition to the contents explicitly stated in this paragraph, any other contents or items not explicitly stated may be directly referred to the contents or items correspondingly stated in the process, without being particularly limited.

The present invention will be further described with reference to the drawings, but the present invention is not limited thereto.

Referring to Fig.1, a hydrocarbon stream 1 and hydrogen 2 are mixed and fed into a hydroconversion reaction zone 3, the hydroconversion reaction zone effluent 4 is fed into a high-pressure separator 5, hydrogen-enriched gas-phase stream 6 obtained by separation is recycled, and the liquid-phase stream is used as an ethylene raw material.

Referring to Fig.2, a hydrocarbon stream 1 and hydrogen 2 are mixed and fed into a hydro-conversion reaction zone 3, the hydro-conversion reaction zone effluent 4 is fed into a high-pressure separator 5, hydrogen-enriched gas-phase stream 6 obtained by separation is recycled, a liquid phase stream 7 is fed into a fractionating column for separation to obtain a gas-phase product 9 serving as an ethylene raw material, and a liquid-phase product 10 is recycled to an inlet of the hydro-conversion reaction zone 3.

### Examples

The present invention will be described in further detail below referring to the Examples and Comparative Examples, but the present invention is not limited to these Examples.

In the following Examples and Comparative Examples, the high-pressure separator 5 was operated at a pressure of 2.5 MPaG and a temperature of 50 °C and the fractionation column was operated at a pressure of 1.0 MPaG and a temperature of 40 °C.

### First series of Examples

In the following Examples and Comparative Examples, the yield was calculated as the mass percent of the output compared to the feed amount, and the hydrogen consumption was calculated as the mass percent of the hydrogen consumption compared to the feed amount.

In the following Examples and Comparative Examples, each hydroconversion catalyst was represented by Cat-A with a number, such as Cat-A1, Cat-A2, Cat-A3, and the like. The hydroconversion catalyst was prepared by a conventional saturation impregnation process for active metal, and the physicochemical properties of the obtained catalyst were shown in Table 1.

Cat-A1 to Cat-A3 were prepared by the process comprising:
(1) selecting mordenite with a SiO₂/Al₂O₃ molar ratio of 15, mechanically mixing the mordenite with a binder, molding, drying at 120 °C for 10 hours, and calcinating at 500 °C for 4 hours, to prepare a catalyst support.
(2) impregnating the catalyst support obtained in step (1) with a solution containing active components, drying at 120 °C for 10 hours, and calcinating at 500 °C for 4 hours, to prepare the hydroconversion catalyst.

Cat-A4 to Cat- A7 were prepared by the process comprising:
(1) selecting ZSM-5 with a SiO₂/Al₂O₃ molar ratio of 10, mechanically mixing the ZSM-5 with a binder, molding, drying at 120°C for 10 hours, and calcinating at 500°C for 4 hours, to prepare a catalyst support.
(2) impregnating the catalyst support obtained in step (1) with a solution containing active components, drying at 120°C for 10 hours, and calcinating at 500°C for 4 hours, to prepare the hydroconversion catalyst.

The properties of raw material were shown in Table 2.

**Table 1**

| Catalyst | Cat-A1 | Cat-A2 | Cat-A3 | Cat-A4 | Cat-A5 | Cat-A6 | Cat-A7 |
|---|---|---|---|---|---|---|---|
| Pore volume, cm³/g | 0.35 | 0.45 | 0.25 | 0.35 | 0.45 | 0.25 | 0.35 |
| Specific surface area, m²/g | 300 | 200 | 400 | 300 | 200 | 400 | 300 |

| Content, wt%, based on the weight of the support | | | | | | | |
|---|---|---|---|---|---|---|---|
| Mordenite | 55 | 40 | 70 | | | | |
| ZSM-5 | | | | 55 | 40 | 70 | 55 |
| Alumina | 19 | 25 | 17 | 19 | 25 | 17 | 44 |

| Active metal content in the catalyst, wt% | | | | | | | |
|---|---|---|---|---|---|---|---|
| MoO₃ | 20 | 25 | 10 | 20 | 25 | 10 | |
| NiO | 6 | 10 | 3 | 6 | 10 | 3 | |
| Pt | | | | | | | 1 |

**Continued Table 1**

| Catalyst | Cat-A8 | Cat-A9 |
|---|---|---|
| Pore volume, cm³/g | 0.35 | 0.35 |
| Specific surface area, m²/g | 300 | 300 |

| Content, wt%, based on the weight of the support | | |
|---|---|---|
| Mordenite | | 40 |
| Y | 55 | |
| Alumina | 19 | 60 |

| Active metal content in the catalyst, wt% | | |
|---|---|---|
| MoO₃ | 20 | 0 |
| NiO | 6 | 0 |

**Table 2**

| Raw material | Raw material 1 | Raw material 2 | Raw material 3 | Raw material 4 | Raw material 5 |
|---|---|---|---|---|---|
| R of the paraffinic alkane | Ethyl | n-propyl | n-butyl | n-pentyl | n-hexyl |

| Raw material | Raw material 6 | Raw material 7 | Raw material 8 | Raw material 9 | Raw material 15 |
|---|---|---|---|---|---|
| R of the paraffinic alkane | Isopropyl | Tert-butyl | Methyl | n-heptyl | Methyl |

**Continued Table 2**

| Raw material | Raw material 10 | Raw material 11 | Raw material 12 | Raw material 13 | Raw material 14 |
|---|---|---|---|---|---|
| 2-methylbutane,% | 72 | 75 | 78 | 65 | 85 |
| 2, 2-dimethylpropane% | 28 | 25 | 22 | 35 | 15 |

### Example 1

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 2

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-A2;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 3

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-A3;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 4

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-A4;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 5

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-A5;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 6

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-A6;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 7

The procedures according to Fig.1 were used for the conversion process, comprising:
(1) The raw material 2 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 8

The procedures according to Fig.1 were used for the conversion process, comprising:
(1) The raw material 2 and hydrogen were mixed and sequentially fed into a hydroconversion reaction zone; which hydroconversion reaction zone was loaded with hydroconversion catalyst Cat-A2;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 9

The procedures according to Fig.1 were used for the conversion process, comprising:
(1) The raw material 2 and hydrogen were mixed and sequentially fed into a hydroconversion reaction zone; which hydroconversion reaction zone was loaded with hydroconversion catalyst Cat-A3;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 10

The procedures according to Fig.1 were used for the conversion process, comprising:
(1) The raw material 2 and hydrogen were mixed and sequentially fed into a hydroconversion reaction zone; which hydroconversion reaction zone was loaded with hydroconversion catalyst Cat-A4;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 11

The procedures according to Fig.1 were used for the conversion process, comprising:
(1) The raw material 2 and hydrogen were mixed and sequentially fed into a hydroconversion reaction zone; which hydroconversion reaction zone was loaded with hydroconversion catalyst Cat-A5;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 12

The procedures according to Fig.1 were used for the conversion process, comprising:
(1) The raw material 2 and hydrogen were mixed and sequentially fed into a hydroconversion reaction zone; which hydroconversion reaction zone was loaded with hydroconversion catalyst Cat-A6;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 13

The procedures according to Fig.1 were used for the conversion process, comprising:
(1) The raw material 3 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 14

The procedures according to Fig.1 were used for the conversion process, comprising:
(1) The raw material 4 and hydrogen were mixed and sequentially fed into a hydroconversion reaction zone; which hydroconversion reaction zone was loaded with hydroconversion catalyst Cat-A4;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 15

The procedures according to Fig.1 were used for the conversion process, comprising:
(1) the raw material 5 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 16

The procedures according to Fig.1 were used for the conversion process, comprising:
(1) the raw material 6 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 17

The procedures according to Fig.1 were used for the conversion process, comprising:
(1) The raw material 7 and hydrogen were mixed and sequentially fed into a hydroconversion reaction zone; which hydroconversion reaction zone was loaded with hydroconversion catalyst Cat-A4;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 18

The procedures according to Fig.1 were used for the conversion process, comprising:
(1) the raw material 10 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 19

The procedures according to Fig.1 were used for the conversion process, comprising:
(1) the raw material 11 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 20

The procedures according to Fig.1 were used for the conversion process, comprising:
(1) the raw material 12 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 21

The procedures according to Fig.1 were used for the conversion process, comprising:
(1) the raw material 13 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Example 22

The procedures according to Fig.1 were used for the conversion process, comprising:
(1) the raw material 14 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Comparative Example 1

The procedures according to Fig.1 were used for the conversion process, comprising:
(1) the raw material 8 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Comparative Example 2

The procedures according to Fig.1 were used for the conversion process, comprising:
(1) the raw material 8 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Comparative Example 3

The procedures according to Fig.1 were used for the conversion process, comprising:
(1) the raw material 9 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Comparative Example 4

The procedures according to Fig.1 were used for the conversion process, comprising:
(1) the raw material 9 and hydrogen were mixed and sequentially fed into a hydroconversion reaction zone; which hydroconversion reaction zone was loaded with hydroconversion catalyst Cat-A4;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Comparative Example 5

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-A8;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Comparative Example 6

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-A9;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Comparative Example 7

The procedures according to Fig.1 were used for the conversion process, comprising:
(1) the raw material 15 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

**Table 3 Quality improvement results**

| No. | Example 1 | Example 2 | Example 3 |
|---|---|---|---|
| Hydroconversion | | | |
| Raw material | Raw material 1 | Raw material 1 | Raw material 1 |
| Catalyst | Cat-A1 | Cat-A2 | Cat-A3 |
| Reaction temperature/°C | 400 | 390 | 405 |
| Reaction pressure/MPa | 4 | 4 | 4 |
| Volume ratio of hydrogen to oil | 800 | 800 | 600 |
| Volume space velocity/h⁻¹ | 1.2 | 1 | 1 |
| Hydrogen consumption, % | 0.9 | 0.77 | 1.44 |
| Content of C₂-C₆ n-alkanes in the product, wt% | 47 | 45 | 62 |

| Preparation of ethylene by steam cracking | | | |
|---|---|---|---|
| Reaction temperature/°C | 880 | 880 | 880 |
| Reaction pressure/MPa | 0.3 | 0.3 | 0.3 |
| Mass ratio of water to oil | 0.4 | 0.4 | 0.4 |
| Yield of ethylene, wt% | 32.7 | 32.1 | 36.5 |
| Yield of propylene, wt% | 15.7 | 15.6 | 15.1 |
| Yield of butadiene, wt% | 3.9 | 4 | 3.4 |

| No. | Example 4 | Example 5 | Example 6 |
|---|---|---|---|
| Hydroconversion | | | |
| Raw material | Raw material 1 | Raw material 1 | Raw material 1 |
| Catalyst | Cat-A4 | Cat-A5 | Cat-A6 |
| Reaction temperature/°C | 390 | 400 | 405 |
| Reaction pressure/MPa | 4 | 4 | 4 |
| Volume ratio of hydrogen to oil | 600 | 600 | 600 |
| Volume space velocity/h⁻¹ | 1 | 0.8 | 1 |
| Hydrogen consumption, % | 1.1 | 1.31 | 1.74 |
| Content of product C2-C6 n-alkanes, wt% | 50 | 53 | 65 |

| Preparation of ethylene by steam cracking | | | |
|---|---|---|---|
| Reaction temperature/°C | 880 | 880 | 880 |
| Reaction pressure/MPa | 0.3 | 0.3 | 0.3 |
| Mass ratio of water to oil | 0.4 | 0.4 | 0.4 |
| Yield of ethylene, wt% | 33.5 | 34.4 | 37.3 |
| Yield of propylene, wt% | 15.5 | 15.3 | 14.9 |
| Yield of butadiene, wt% | 3.8 | 3.7 | 3.3 |

**Continued Table 3 Quality improvement results**

| No. | Example 7 | Example 8 | Example 9 |
|---|---|---|---|
| Hydroconversion | | | |
| Raw material | Raw material 2 | Raw material 2 | Raw material 2 |
| Catalyst | Cat-A1 | Cat-A2 | Cat-A3 |
| Reaction temperature/°C | 380 | 390 | 395 |
| Reaction pressure/MPa | 4 | 4 | 4 |
| Volume ratio of hydrogen to oil | 600 | 600 | 600 |
| Volume space velocity/h⁻¹ | 1 | 0.8 | 1 |
| Hydrogen consumption, % | 0.94 | 1.25 | 1.54 |
| Content of C₂-C₆ n-alkanes in the product, wt% | 44 | 49 | 61 |

| Preparation of ethylene by steam cracking | | | |
|---|---|---|---|
| Reaction temperature/°C | 880 | 880 | 880 |
| Reaction pressure/MPa | 0.3 | 0.3 | 0.3 |
| Mass ratio of water to oil | 0.4 | 0.4 | 0.4 |
| Yield of ethylene, wt% | 31.8 | 33.4 | 36.2 |
| Yield of propylene, wt% | 15.6 | 15.6 | 15.1 |
| Yield of butadiene, wt% | 4 | 3.8 | 3.4 |

| No. | Example 10 | Example 11 | Example 12 |
|---|---|---|---|
| Hydroconversion | | | |
| Raw material | Raw material 2 | Raw material 2 | Raw material 2 |
| Catalyst | Cat-A4 | Cat-A5 | Cat-A6 |
| Reaction temperature/°C | 390 | 395 | 400 |
| Reaction pressure/MPa | 4 | 4 | 4 |
| Volume ratio of hydrogen to oil | 600 | 600 | 600 |
| Volume space velocity/h-1 | 1 | 1 | 1 |
| Hydrogen consumption, % | 1.1 | 1.51 | 2 |
| Content of product C2-C6 n-alkanes, wt% | 45 | 59 | 66 |

| Preparation of ethylene by steam cracking | | | |
|---|---|---|---|
| Reaction temperature/°C | 880 | 880 | 880 |
| Reaction pressure/MPa | 0.3 | 0.3 | 0.3 |
| Mass ratio of water to oil | 0.4 | 0.4 | 0.4 |
| Yield of ethylene, wt% | 32.1 | 35.6 | 37.6 |
| Yield of propylene, wt% | 15.6 | 15.2 | 14.9 |
| Yield of butadiene, wt% | 4 | 3.4 | 3.3 |

**Table 3 quality improvement results**

| No. | Example 13 | Example 14 | Example 15 |
|---|---|---|---|
| Hydroconversion | | | |
| Raw material | Raw material 3 | Raw material 4 | Raw material 5 |
| Catalyst | Cat-A1 | Cat-A4 | Cat-A1 |
| Reaction temperature/°C | 370 | 360 | 350 |
| Reaction pressure/MPa | 4 | 4 | 4 |
| Volume ratio of hydrogen to oil | 600 | 600 | 600 |
| Volume space velocity/h⁻¹ | 1 | 1 | 1 |
| Hydrogen consumption, % | 1.14 | 1.23 | 1.34 |
| Content of C₂-C₆ n-alkanes in the product, wt% | 40 | 39 | 38 |

| Preparation of ethylene by steam cracking | | | |
|---|---|---|---|
| Reaction temperature/°C | 880 | 880 | 880 |
| Reaction pressure/MPa | 0.3 | 0.3 | 0.3 |
| Mass ratio of water to oil | 0.4 | 0.4 | 0.4 |
| Yield of ethylene, wt% | 30.6 | 30.3 | 29.9 |
| Yield of propylene, wt% | 15.9 | 15.9 | 15.9 |
| Yield of butadiene, wt% | 4.2 | 4.2 | 4.2 |

| No. | Example 16 | Example 17 | Example 18 |
|---|---|---|---|
| Hydroconversion | | | |
| Raw material | Raw material 6 | Raw material 7 | Raw material 10 |
| Catalyst | Cat-A1 | Cat-A4 | Cat-A1 |
| Reaction temperature/°C | 400 | 400 | 400 |
| Reaction pressure/MPa | 4 | 4 | 4 |
| Volume ratio of hydrogen to oil | 600 | 800 | 800 |
| Volume space velocity/h⁻¹ | 0.8 | 1 | 1.2 |
| Hydrogen consumption, % | 1.2 | 1.24 | 0.9 |
| Content of C₂-C₆ n-alkanes in the product, wt% | 35 | 36 | 40 |

| Preparation of ethylene by steam cracking | | | |
|---|---|---|---|
| Reaction temperature/°C | 880 | 880 | 880 |
| Reaction pressure/MPa | 0.3 | 0.3 | 0.3 |
| Mass ratio of water to oil | 0.4 | 0.4 | 0.4 |
| Yield of ethylene, wt% | 28.9 | 29.3 | 30.8 |
| Yield of propylene, wt% | 16.1 | 16.1 | 16.0 |
| Yield of butadiene, wt% | 4.3 | 4.3 | 4.0 |

| No. | Example 19 | Example 20 | Example 21 |
|---|---|---|---|
| Hydroconversion | | | |
| Raw material | Raw material 11 | Raw material 12 | Raw material 13 |
| Catalyst | Cat-A1 | Cat-A1 | Cat-A1 |
| Reaction temperature/°C | 400 | 400 | 400 |
| Reaction pressure/MPa | 4 | 4 | 4 |
| Volume ratio of hydrogen to oil | 800 | 800 | 800 |
| Volume space velocity/h⁻¹ | 1.2 | 1.2 | 1.2 |
| Hydrogen consumption, % | 0.9 | 0.9 | 0.9 |
| Content of C₂-C₆ n-alkanes in the product, wt% | 41 | 42 | 37 |

| Preparation of ethylene by steam cracking | | | |
|---|---|---|---|
| Reaction temperature/°C | 880 | 880 | 880 |
| Reaction pressure/MPa | 0.3 | 0.3 | 0.3 |
| Mass ratio of water to oil | 0.4 | 0.4 | 0.4 |
| Yield of ethylene, wt% | 31.3 | 31.9 | 29.2 |
| Yield of propylene, wt% | 15.9 | 15.8 | 16.3 |
| Yield of butadiene, wt% | 4.0 | 3.9 | 4.1 |

| No. | Example 22 | | |
|---|---|---|---|
| Hydroconversion | | | |
| Raw material | Raw material 14 | | |
| Catalyst | Cat-A1 | | |
| Reaction temperature/°C | 400 | | |
| Reaction pressure/MPa | 4 | | |
| Volume ratio of hydrogen to oil | 800 | | |
| Volume space velocity/h⁻¹ | 1.2 | | |
| Hydrogen consumption, % | 0.9 | | |
| Content of C₂-C₆ n-alkanes in the product, wt% | 44 | | |

| Preparation of ethylene by steam cracking | | | |
|---|---|---|---|
| Reaction temperature/°C | 880 | | |
| Reaction pressure/MPa | 0.3 | | |
| Mass ratio of water to oil | 0.4 | | |
| Yield of ethylene, wt% | 31.4 | | |
| Yield of propylene, wt% | 15.8 | | |
| Yield of butadiene, wt% | 3.9 | | |

**Continued Table 3 Quality improvement results**

| No. | Comparative Example 1 | Comparative Example 2 | Comparative Example 3 | Comparative Example 4 |
|---|---|---|---|---|
| Hydroconversion | | | | |
| Raw material | Raw material 8 | Raw material 8 | Raw material 9 | Raw material 9 |
| Catalyst | Cat-A1 | Cat-A4 | Cat-A1 | Cat-A4 |
| Reaction temperature/°C | 410 | 400 | 350 | 350 |
| Reaction pressure/MPa | 4 | 4 | 4 | 4 |
| Volume ratio of hydrogen to oil | 400 | 600 | 600 | 600 |
| Volume space velocity/h⁻¹ | 0.6 | 0.6 | 1 | 1 |
| Hydrogen consumption, % | 0.84 | 1.23 | 1.54 | 1.63 |
| Content of C₂-C₆ n-alkanes in the product, wt% | 26 | 27 | 28 | 29 |

| Preparation of ethylene by steam cracking | | | | |
|---|---|---|---|---|
| Reaction temperature/°C | 880 | 880 | 880 | 880 |
| Reaction pressure/MPa | 0.3 | 0.3 | 0.3 | 0.3 |
| Mass ratio of water to oil | 0.4 | 0.4 | 0.4 | 0.4 |
| Yield of ethylene, wt% | 25.9 | 26.2 | 26.5 | 26.9 |
| Yield of propylene, wt% | 18.4 | 18.4 | 18.3 | 18.3 |
| Yield of butadiene, wt% | 4.4 | 4.4 | 4.4 | 4.3 |

| No. | Comparative Example 5 | Comparative Example 6 | Comparative Example 7 | |
|---|---|---|---|---|
| Hydroconversion | | | | |
| Raw material | Raw material 1 | Raw material 1 | Raw material 15 | |
| Catalyst | Cat-A8 | Cat-A9 | Cat-A1 | |
| Reaction temperature/°C | 400 | 400 | 400 | |
| Reaction pressure/MPa | 4 | 4 | 4 | |
| Volume ratio of hydrogen to oil | 400 | 600 | 800 | |
| Volume space velocity/h⁻¹ | 0.8 | 0.8 | 1.2 | |
| Hydrogen consumption, % | 0.25 | 0.15 | 0.9 | |
| Content of C₂-C₆ n-alkanes in the product, wt% | 12 | 10 | 36 | |
| Preparation of ethylene by steam cracking | | | | |
| Reaction temperature/°C | 880 | 880 | 880 | |
| Reaction pressure/MPa | 0.3 | 0.3 | 0.3 | |
| Mass ratio of water to oil | 0.4 | 0.4 | 0.4 | |
| Yield of ethylene, wt% | 20.2 | 18.2 | 26.7 | |
| Yield of propylene, wt% | 18.4 | 19.5 | 19.0 | |
| Yield of butadiene, wt% | 4.6 | 4.8 | 4.2 | |

By comparison, it could be seen that the process according to the present invention could effectively convert a low-quality ethylene raw material into a high-quality ethylene raw material, where the hydrocarbon stream after the hydro-conversion could greatly improve the yield of ethylene and the yield of triene of the ethylene device. Moreover, the process procedures according to the present invention was simple with low difficulty of operation, thus had very good economic benefit.

### Second series of Example

In the following Examples and Comparative Examples, the yield was calculated as the mass percent of the output compared to the feed amount.

The processing benefit per ton oil denoted the gain per 1 ton of feedstock processed, calculated as (price of ethylene product-price of feedstock and hydrogen-energy consumption of processing)/processing capacity, taking the benefit obtained by using the feedstock without hydroconversion directly as the ethylene raw material as the reference of the processing benefit per ton oil.

In the following Examples and Comparative Examples, each hydroconversion catalyst was represented by Cat-A, Cat-B, Cat-C with a number, such as Cat- A1, Cat-A2, Cat-A3, and the like. The hydroconversion catalyst was prepared by a conventional saturation impregnation process for active metal, and the physicochemical properties of the obtained catalyst were shown in Table 1.

The catalyst of Cat-A was prepared by the process comprising:
(1) selecting mordenite with a SiO₂/Al₂O₃ molar ratio of 15, mechanically mixing the mordenite with a binder, molding, drying at 120^{°}C for 10 hours, and calcinating at 500^{°}C for 4 hours, to prepare a catalyst support.
(2) impregnating the catalyst support obtained in step (1) with a solution containing active components, drying at 120^{°}C for 10 hours, and calcinating at 500^{°}C for 4 hours, to prepare the hydroconversion catalyst.

The catalyst of Cat-B was prepared by the process comprising:
(1) selecting ZSM-5 molecular sieve with a SiO₂/Al₂O₃ molar ratio of 10, mechanically mixing the ZSM-5 with a binder, molding, drying at 120^{°}C for 10 hours, and calcinating at 500^{°}C for 4 hours, to prepare a catalyst support.
(2) impregnating the catalyst support obtained in step (1) with a solution containing active components, drying at 120^{°}C for 10 hours, and calcinating at 500^{°}C for 4 hours, to prepare the hydroconversion catalyst.

The catalyst of Cat-C was prepared by the process comprising:
(1) selecting ZSM-35 molecular sieve with a SiO₂/Al₂O₃ molar ratio of 20, mechanically mixing the ZSM-35 with a binder, molding, drying at 120^{°}C for 10 hours, and calcinating at 500^{°}C for 4 hours, to prepare a catalyst support.
(2) impregnating the catalyst support obtained in step (1) with a solution containing active components, drying at 120^{°}C for 10 hours, and calcinating at 500^{°}C for 4 hours, to prepare the hydroconversion catalyst.

The catalyst of Cat-D was prepared by the process comprising:
(1) selecting SAPO-34 molecular sieve with a SiO₂/Al₂O₃ molar ratio of 0.5, mechanically mixing the SAPO-34 with a binder, molding, drying at 120^{°}C for 10 hours, and calcinating at 500^{°}C for 4 hours, to prepare a catalyst support.
(2) impregnating the catalyst support obtained in step (1) with a solution containing active components, drying at 120^{°}C for 10 hours, and calcinating at 500^{°}C for 4 hours, to prepare the hydroconversion catalyst.

The catalyst of Cat -E was prepared by the process comprising:
(1) selecting Y molecular sieve with a SiO₂/Al₂O₃ molar ratio of 15, mechanically mixing the Y molecular sieve with a binder, molding, drying at 120^{°}C for 10 hours, and calcinating at 500^{°}C for 4 hours, to prepare a catalyst support.
(2) impregnating the catalyst support obtained in step (1) with a solution containing active components, drying at 120^{°}C for 10 hours, and calcinating at 500^{°}C for 4 hours, to prepare the hydroconversion catalyst.

The catalyst of Cat -F was prepared by the process comprising:
(1) selecting mordenite with a SiO₂/Al₂O₃ molar ratio of 15, mechanically mixing the mordenite with a binder, molding, drying at 120^{°}C for 10 hours, and calcinating at 500^{°}C for 4 hours, to prepare a catalyst support.
(2) Selecting ZSM-5 molecular sieve with a SiO₂/Al₂O₃ molar ratio of 10, mechanically mixing the ZSM-5 with a binder, molding, drying at 120^{°}C for 10 hours, and calcinating at 500^{°}C for 4 hours, to prepare a catalyst support.
(3) impregnating the catalyst supports obtained in steps (1) and (2) with a solution containing active components, drying at 120^{°}C for 10 hours, and calcinating at 500^{°}C for 4 hours, to prepare the hydroconversion catalyst.

The catalyst of Cat-G was prepared by the process comprising:
(1) selecting mordenite with a SiO₂/Al₂O₃ molar ratio of 15, mechanically mixing the mordenite with a binder, molding, drying at 120^{°}C for 10 hours, and calcinating at 500^{°}C for 4 hours, to prepare a catalyst.

The properties of raw materials were shown in Table 2, wherein, the raw material 5 needed to be obtained by adsorption separation or rectification for a raw material containing iso-alkanes.

**Table 1**

| Catalyst | Cat-A1 | Cat-A2 | Cat-A3 | Cat-B1 | Cat-B2 | Cat-B3 | Cat-A4 |
|---|---|---|---|---|---|---|---|
| Pore volume, cm³/g | 0.35 | 0.45 | 0.25 | 0.35 | 0.45 | 0.25 | 0.35 |
| Specific surface area, m²/g | 300 | 200 | 400 | 300 | 200 | 400 | 300 |

| Content, wt%, based on the weight of the support | | | | | | | |
|---|---|---|---|---|---|---|---|
| Mordenite | 55 | 40 | 70 | | | | |
| ZSM-5 | | | | 55 | 40 | 70 | 55 |
| Alumina | 19 | 25 | 17 | 19 | 25 | 17 | 44 |

| Active metal content in the catalyst, wt% | | | | | | | |
|---|---|---|---|---|---|---|---|
| MoO₃ | 20 | 25 | 10 | 20 | 25 | 10 | |
| NiO | 6 | 10 | 3 | 6 | 10 | 3 | |
| Pt | | | | | | | 1 |

**Continued Table 1**

| Catalyst | Cat-C | Cat-D | Cat-E | Cat-G |
|---|---|---|---|---|
| Pore volume, cm³/g | 0.35 | 0.35 | 0.35 | 0.35 |
| Specific surface area, m²/g | 300 | 300 | 300 | 300 |

| Content, wt%, based on the weight of the support | | | | |
|---|---|---|---|---|
| Mordenite | | | | 55 |
| SAPO-14 | 55 | | | |
| ZSM-35 | | 55 | | |
| Y | | | 55 | |
| Alumina | 19 | 19 | 19 | 45 |

| Active metal content in the catalyst, wt% | | | | |
|---|---|---|---|---|
| MoO₃ | 20 | 20 | 20 | 0 |
| NiO | 6 | 6 | 6 | 0 |

**Continued Table 1**

| Catalyst | Cat-F1 | Cat-F2 | Cat-F3 | Cat-F4 |
|---|---|---|---|---|
| Pore volume, cm³/g | 0.38 | 0.37 | 0.39 | 0.38 |
| Specific surface area, m²/g | 350 | 360 | 340 | 350 |

| Content, wt%, based on the weight of the support | | | | |
|---|---|---|---|---|
| Mordenite | 30 | 12 | 48 | 42 |
| ZSM-5 | 30 | 48 | 12 | 18 |
| Alumina | 15 | 15 | 15 | 15 |

| Active metal content in the catalyst, wt% | | | | |
|---|---|---|---|---|
| MoO₃ | 20 | 20 | 20 | 20 |
| NiO | 5 | 5 | 5 | 5 |

**Table 2**

| Raw material | Raw material 1 | Raw material 2 | Raw material 3 | Raw material 4 | Raw material 5 | Raw material 6 |
|---|---|---|---|---|---|---|
| C₄ iso-alkane content, wt% | 1 | 2 | 0.5 | 1 | 0.4 | 80 |
| C₄ n-alkane content, wt% | 2 | 3 | 1.5 | 2 | 0.6 | 11 |
| C₅-C₆ n-alkanes content, wt% | 16 | 28 | 10 | 42 | 3 | 2 |
| C₅-C₆ iso-alkanes content, wt% | 78 | 65 | 85 | 50 | 95 | 7 |
| C₇₊ Hydrocarbons and Cyclic Hydrocarbon | 3 | 2 | 3 | 5 | 1 | 0 |
| Initial boiling point, °C | 24 | 25 | 22 | 25 | 23 | 16 |
| End boiling point, °C | 64 | 66 | 63 | 67 | 62 | 45 |

### Example 1

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, about 33 wt% of the total amount of C₅-C₆ iso-alkanes was converted to C₂-C₆ n-alkanes, and the weight ratio of C₂-C₃ hydrocarbons to C₄-C₆ n-alkanes in the reaction effluent was 0.9:1.

### Example 2

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-A2;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, about 37 wt% of the total amount of C₅-C₆ iso-alkanes was converted to C₂-C₆ n-alkanes, and the weight ratio of C₂-C₃ hydrocarbons to C₄-C₆ n-alkanes in the reaction effluent was 1.4:1.

### Example 3

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-A3;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, about 42wt% of the total amount of C₅-C₆ iso-alkanes was converted to C₂-C₆ n-alkanes, and the weight ratio of C₂-C₃ hydrocarbons to C₄-C₆ n-alkanes in the reaction effluent was 2.1:1.

### Example 4

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-B1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, about 62wt% of the total amount of C₅-C₆ iso-alkanes was converted to C₂-C₆ n-alkanes, and the weight ratio of C₂-C₃ hydrocarbons to C₄-C₆ n-alkanes in the reaction effluent was 5:1.

### Example 5

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-B2;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, about 57wt% of the total amount of C₅-C₆ iso-alkanes was converted to C₂-C₆ n-alkanes, and the weight ratio of C₂-C₃ hydrocarbons to C₄-C₆ n-alkanes in the reaction effluent was 4.4:1.

### Example 6

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-B3;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, about 52wt% of the total amount of C₅-C₆ iso-alkanes was converted to C₂-C₆ n-alkanes, and the weight ratio of C₂-C₃ hydrocarbons to C₄-C₆ n-alkanes in the reaction effluent was 3.9:1.

### Example 7

The procedures according to Fig.1 were used for the conversion process, comprising:
(1) the raw material 1 and hydrogen were mixed and sequentially fed into a hydroconversion reaction zone; which hydroconversion reaction zone was loaded, along the flowing direction of the raw material, successively with hydroconversion catalysts Cat-B1 and Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, about 48wt% of the total amount of C₅-C₆ iso-alkanes was converted to C₂-C₆ n-alkanes, and the weight ratio of C₂-C₃ hydrocarbons to C₄-C₆ n-alkanes in the reaction effluent was 3.2:1.

### Example 8

The procedures according to Fig.1 were used for the conversion process, comprising:
(1) the raw material 1 and hydrogen were mixed and sequentially fed into a hydroconversion reaction zone; which hydroconversion reaction zone was loaded, along the flowing direction of the raw material, successively with hydroconversion catalysts Cat-B1 and Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, about 44wt% of the total amount of C₅-C₆ iso-alkanes was converted to C₂-C₆ n-alkanes, and the weight ratio of C₂-C₃ hydrocarbons to C₄-C₆ n-alkanes in the reaction effluent was 2.3:1.

### Example 9

The procedures according to Fig.1 were used for the conversion process, comprising:
(1) the raw material 1 and hydrogen were mixed and sequentially fed into a hydroconversion reaction zone; which hydroconversion reaction zone was loaded, along the flowing direction of the raw material, successively with hydroconversion catalysts Cat-B1 and Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, about 40wt% of the total amount of C₅-C₆ iso-alkanes was converted to C₂-C₆ n-alkanes, and the weight ratio of C₂-C₃ hydrocarbons to C₄-C₆ n-alkanes in the reaction effluent was 2.5:1.

### Example 10

The procedures according to Fig.1 were used for the conversion process, comprising:
(1) the raw material 1 and hydrogen were mixed and sequentially fed into a hydroconversion reaction zone; which hydroconversion reaction zone was loaded, along the flowing direction of the raw material, successively with hydroconversion catalysts Cat-B1 and Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, about 38wt% of the total amount of C₅-C₆ iso-alkanes was converted to C₂-C₆ n-alkanes, and the weight ratio of C₂-C₃ hydrocarbons to C₄-C₆ n-alkanes in the reaction effluent was 1.8:1.

### Example 11

The procedures according to Fig.1 were used for the conversion process, comprising:
(1) the raw material 1 and hydrogen were mixed and sequentially fed into a hydroconversion reaction zone; which hydroconversion reaction zone was loaded, along the flowing direction of the raw material, successively with hydroconversion catalysts Cat-B1 and Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, about 36wt% of the total amount of C₅-C₆ iso-alkanes was converted to C₂-C₆ n-alkanes, and the weight ratio of C₂-C₃ hydrocarbons to C₄-C₆ n-alkanes in the reaction effluent was 1.3:1.

### Example 12

The procedures according to Fig.1 were used for the conversion process, comprising:
(1) The raw material 2 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, about 42wt% of the total amount of C₅-C₆ iso-alkanes was converted to C₂-C₆ n-alkanes, and the weight ratio of C₂-C₃ hydrocarbons to C₄-C₆ n-alkanes in the reaction effluent was 2.2:1.

### Example 13

The procedures according to Fig.1 were used for the conversion process, comprising:
(1) the raw material 3 and hydrogen were mixed and sequentially fed into a hydroconversion reaction zone; which hydroconversion reaction zone was loaded with hydroconversion catalyst Cat-B1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, about 56wt% of the total amount of C₅-C₆ iso-alkanes was converted to C₂-C₆ n-alkanes, and the weight ratio of C₂-C₃ hydrocarbons to C₄-C₆ n-alkanes in the reaction effluent was 4.1:1.

### Example 14

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, about 25wt% of the total amount of C₅-C₆ iso-alkanes was converted to C₂-C₆ n-alkanes, and the weight ratio of C₂-C₃ hydrocarbons to C₄-C₆ n-alkanes in the reaction effluent was 0.7:1.

### Example 15

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-C;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, about 28wt% of the total amount of C₅-C₆ iso-alkanes was converted to C₂-C₆ n-alkanes, and the weight ratio of C₂-C₃ hydrocarbons to C₄-C₆ n-alkanes in the reaction effluent was 0.8:1.

### Example 16

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-D;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, about 26wt% of the total amount of C₅-C₆ iso-alkanes was converted to C₂-C₆ n-alkanes, and the weight ratio of C₂-C₃ hydrocarbons to C₄-C₆ n-alkanes in the reaction effluent was 0.7:1.

### Example 17

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-F1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

### Example 18

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-F2;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

### Example 19

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-F3;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

### Example 20

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-F4;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

### Example 21

The process for upgrading a hydrocarbon stream used the scheme as shown in Fig.2, comprising:
(1) the raw material 1 and hydrogen were mixed and sequentially fed into a hydroconversion reaction zone; which hydroconversion reaction zone was loaded with hydroconversion catalyst Cat-B1;
(2) the reaction effluent from the hydroconversion reaction zone of step (1) was subjected to gas-liquid separation to obtain a hydrogen-rich gas and a liquid-phase stream, recycling the hydrogen-rich gas, separating the liquid-phase stream to obtain C₂ and C₃ serving as ethylene raw materials, and cracking the rest stream (called as C₄-C₆ recycled stream). The process conditions and the quality improvement results in this Example were shown in Table 3.

In this Example, about 78 wt% of the total amount of C₅-C₆ iso-alkanes was converted into C₂-C₃ n-alkanes. The weight ratio of C₄-C₆ recycled stream to the raw material 1 was 0.3:1.

### Example 22

The process for upgrading a hydrocarbon stream used the scheme as shown in Fig.2, comprising:
(1) the raw material 1 and hydrogen were mixed and sequentially fed into a hydroconversion reaction zone; which hydroconversion reaction zone was loaded with hydroconversion catalyst Cat-B2;
(2) the reaction effluent from the hydroconversion reaction zone of step (1) was subjected to gas-liquid separation to obtain a hydrogen-rich gas and a liquid-phase stream, recycling the hydrogen-rich gas, separating the liquid-phase stream to obtain C₂ and C₃ serving as ethylene raw materials, and recycling and cracking the C₄-C₆.

The process conditions and the quality improvement results in this Example were shown in Table 3.

In this Example, about 78 wt% of the total amount of C₅-C₆ iso-alkanes was converted into C₂-C₃ n-alkanes. The weight ratio of C₄-C₆ recycled stream to the raw material 1 was 0.4:1.

### Example 23

The process for upgrading a hydrocarbon stream used the scheme as shown in Fig.2, comprising:
(1) the raw material 1 and hydrogen were mixed and sequentially fed into a hydroconversion reaction zone; which hydroconversion reaction zone was loaded with hydroconversion catalyst Cat-B3;
(2) the reaction effluent from the hydroconversion reaction zone of step (1) was subjected to gas-liquid separation to obtain a hydrogen-rich gas and a liquid-phase stream, recycling the hydrogen-rich gas, separating the liquid-phase stream to obtain C₂ and C₃ serving as ethylene raw materials, and recycling and cracking the C₄-C₆.

The process conditions and the quality improvement results in this Example were shown in Table 3.

In this Example, about 78 wt% of the total amount of C₅-C₆ iso-alkanes was converted into C₂-C₃ n-alkanes. The weight ratio of C₄-C₆ recycled stream to the raw material 1 was 0.3:1.

### Comparative Example 1

The procedures according to Fig.1 were used for the conversion process, comprising:
(1) the raw material 4 and hydrogen were mixed and sequentially fed into a hydroconversion reaction zone; which hydroconversion reaction zone was loaded with hydroconversion catalyst Cat-A1;
2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

### Comparative Example 2

The procedures according to Fig.1 were used for the conversion process, comprising:
(1) the raw material 4 and hydrogen were mixed and sequentially fed into a hydroconversion reaction zone; which hydroconversion reaction zone was loaded with hydroconversion catalyst Cat-B1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

### Comparative Example 3

The procedures according to Fig.1 were used for the conversion process, comprising:
(1) the raw material 5 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

### Comparative Example 4

The procedures according to Fig.1 were used for the conversion process, comprising:
(1) the raw material 5 and hydrogen were mixed and sequentially fed into a hydroconversion reaction zone; which hydroconversion reaction zone was loaded with hydroconversion catalyst Cat-B1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

### Comparative Example 5

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-A4;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

### Comparative Example 6

The procedures according to Fig.1 were used for the conversion process, comprising:
(1) the raw material 6 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

### Comparative Example 7

The procedures according to Fig.1 were used for the conversion process, comprising:
(1) the raw material 1 and hydrogen were mixed and sequentially fed into a hydroconversion reaction zone; which hydroconversion reaction zone was loaded with hydroconversion catalyst Cat-E;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

### Comparative Example 8

The procedures according to Fig.1 were used for the chemical conversion process, comprising:
(1) the raw material 1 and hydrogen were mixed and sequentially fed into a hydroconversion reaction zone; which hydroconversion reaction zone was loaded with hydroconversion catalyst Cat-A4;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

### Comparative Example 9

The procedures according to Fig.1 were used, comprising:
(1) the raw material 1 and hydrogen were mixed and sequentially fed into a hydroconversion reaction zone, with a reaction temperature of 250 °C ; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Comparative Example 10

The procedures according to Fig.1 were used, comprising:
(1) the raw material 1 and hydrogen were mixed and sequentially fed into a hydroconversion reaction zone, with a reaction temperature of 200 °C ; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Comparative Example 11

The procedures according to Fig.1 were used, comprising:
(1) the raw material 1 and hydrogen were mixed and sequentially fed into a hydroconversion reaction zone, with a volume ratio of hydrogen to oil of 20:1; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Comparative Example 12

The procedures according to Fig.1 were used, comprising:
(1) the raw material 1 and hydrogen were mixed and sequentially fed into a hydroconversion reaction zone, with a volume ratio of hydrogen to oil of 5:1; which hydroconversion reaction zone was loaded with hydroconversion catalyst Cat-A1;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Comparative Example 13

The procedures according to Fig.1 were used, comprising:
(1) the raw material 1 and hydrogen were mixed and sequentially fed into a hydroconversion reaction zone; which hydroconversion reaction zone was loaded with commercially available solid acid catalyst;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the quality improvement results in this Example were shown in Table 3.

### Comparative Example 14

The procedures according to Fig.1 were used, comprising:
(1) the raw material 1 and hydrogen were mixed and sequentially fed into a hydroconversion reaction zone; which hydroconversion reaction zone was loaded with hydroconversion catalyst Cat-G;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

### Comparative Example 15

The procedures according to Fig.1 were used, comprising:
(1) the raw material 1 and hydrogen were mixed and sequentially fed into a hydroconversion reaction zone; which hydroconversion reaction zone was loaded with hydroconversion catalyst Cat-A1;
(2) the reaction effluent from the hydroconversion reaction zone of step (1) was subjected to gas-liquid separation to obtain a hydrogen-rich gas and a liquid-phase stream, recycling the hydrogen-rich gas, separating the liquid-phase stream for n- and iso-alkanes, from which the n-alkanes were used as ethylene raw materials, and the isoalkanes were recycled to the hydroconversion reaction zone. The process conditions and the conversion results in this Example were shown in Table 3.

**Table 3 Conversion results**

| No. | Example 1 | Example 2 | Example 3 |
|---|---|---|---|
| Hydroconversion | | | |
| Raw material | Raw material 1 | Raw material 1 | Raw material 1 |
| Catalyst | Cat-A1 | Cat-A2 | Cat-A3 |
| Reaction temperature/°C | 400 | 400 | 405 |
| Reaction pressure/MPa | 4 | 4 | 4 |
| Volume ratio of hydrogen to oil | 600 | 500 | 600 |
| Volume space velocity/h⁻¹ | 1.2 | 0.8 | 1 |
| Content of C₂-C₆ n-alkanes in the product, wt% | 49 | 53 | 58 |

| Preparation of ethylene by steam cracking | | | |
|---|---|---|---|
| Reaction temperature/°C | 880 | 880 | 880 |
| Reaction pressure/MPa | 0.3 | 0.3 | 0.3 |
| Mass ratio of water to oil | 0.4 | 0.4 | 0.4 |
| Yield of ethylene, wt% | 32.8 | 33.2 | 35.4 |
| Yield of propylene, wt% | 17.7 | 17.4 | 16.6 |
| Yield of butadiene, wt% | 3.8 | 3.8 | 3.7 |
| Processing benefit per ton oil, CNY/ton | Reference+190 | Reference+170 | Reference+160 |

| No. | Example 4 | Example 5 | Example 6 |
|---|---|---|---|
| Hydroconversion | | | |
| Raw material | Raw material 1 | Raw material 1 | Raw material 1 |
| Catalyst | Cat-B1 | Cat-B2 | Cat-B3 |
| Reaction temperature/°C | 420 | 410 | 410 |
| Reaction pressure/MPa | 4 | 4 | 4 |
| Volume ratio of hydrogen to oil | 500 | 400 | 400 |
| Volume space velocity/h-1 | 0.8 | 1 | 0.8 |
| Content of product C2-C6 n-alkanes, wt% | 78 | 73 | 68 |

| Preparation of ethylene by steam cracking | | | |
|---|---|---|---|
| Reaction temperature/°C | 880 | 880 | 880 |
| Reaction pressure/MPa | 0.3 | 0.3 | 0.3 |
| Mass ratio of water to oil | 0.4 | 0.4 | 0.4 |
| Yield of ethylene, wt% | 40.2 | 39.5 | 37.9 |
| Yield of propylene, wt% | 13.5 | 13.8 | 14.5 |
| Yield of butadiene, wt% | 3.3 | 3.4 | 3.6 |
| Processing benefit per ton oil, CNY/ton | Reference+190 | Reference+180 | Reference+160 |

**Continued Table 3 Conversion results**

| No. | Example 7 | Example 8 | Exampl e 9 | Example 10 | Example 11 |
|---|---|---|---|---|---|
| Hydroconversion | | | | | |
| Raw material | Raw material 1 | Raw material 1 | Raw materia l 1 | Raw material 1 | Raw material 1 |
| Catalyst | Cat-B1/Cat-A1 | Cat-B1/Cat-A1 | Cat-B1/Cat -A1 | Cat-B1/Cat-A1 | Cat-B1/Cat-A1 |
| Weight ratio of catalyst A to catalyst B | 1:5 | 1: 2 | 1:1 | 2,1 | 5:1 |
| Reaction temperature/°C | 405 | 400 | 400 | 390 | 390 |
| Reaction pressure/MPa | 4 | 4 | 4 | 4 | 4 |
| Volume ratio of hydrogen to oil | 400 | 500 | 600 | 500 | 600 |
| Volume space velocity/h⁻¹ | 0.8 | 0.8 | 0.8 | 0.8 | 0.6 |
| Content of C₂-C₆ n-alkanes in the product, wt% | 64 | 60 | 56 | 54 | 52 |

| Preparation of ethylene by steam cracking | | | | | |
|---|---|---|---|---|---|
| Reaction temperature/°C | 880 | 880 | 880 | 880 | 880 |
| Reaction pressure/MPa | 0.3 | 0.3 | 0.3 | 0.3 | 0.3 |
| Mass ratio of water to oil | 0.4 | 0.4 | 0.4 | 0.4 | 0.4 |
| Yield of ethylene, wt% | 37.2 | 36.1 | 34.6 | 33.8 | 33.2 |
| Yield of propylene, wt% | 15.7 | 16.4 | 16.8 | 17.7 | 17.9 |
| Yield of butadiene, wt% | 3.5 | 3.6 | 3.7 | 3.8 | 3.9 |
| Processing benefit per ton oil, CNY/ton | Reference +160 | Referenc e+170 | Refere nce+19 0 | Reference +170 | Reference +150 |

| No. | Example 12 | Example 13 | Exampl e 14 | Example 15 | Example 16 |
|---|---|---|---|---|---|
| Hydroconversion | | | | | |
| Raw material | Raw material 2 | Raw material 3 | Raw materia 1 | Raw material 1 | Raw material 1 |
| Catalyst | Cat-A1 | Cat-A4 | Cat-A1 | Cat-C1 | Cat-D1 |
| Reaction temperature/°C | 400 | 405 | 380 | 400 | 400 |
| Reaction pressure/MPa | 4 | 4 | 4 | 4 | 4 |
| Volume ratio of hydrogen to oil | 400 | 400 | 400 | 600 | 600 |
| Volume space velocity/h-1 | 0.8 | 0.8 | 1.1 | 1.2 | 1.2 |
| Content of product C2-C6 n-alkanes, wt% | 58 | 72 | 41 | 44 | 42 |

| Preparation of ethylene by steam cracking | | | | | |
|---|---|---|---|---|---|
| Reaction temperature/°C | 880 | 880 | 880 | 880 | 880 |
| Reaction pressure/MPa | 0.3 | 0.3 | 0.3 | 0.3 | 0.3 |
| Mass ratio of water to oil | 0.4 | 0.4 | 0.4 | 0.4 | 0.4 |
| Yield of ethylene, wt% | 35.2 | 39.2 | 29.8 | 31.2 | 30.5 |
| Yield of propylene, wt% | 16.6 | 13.8 | 19.2 | 18.6 | 19.3 |
| Yield of butadiene, wt% | 3.8 | 3.4 | 4 | 3.9 | 4 |
| Processing benefit per ton oil, CNY/ton | Reference +70 | Referenc e+80 | Refere nce+50 | Reference +60 | Reference +50 |

| No. | Example 17 | Example 18 | Exampl e 19 | Example 20 | Example 21 |
|---|---|---|---|---|---|
| Hydroconversion | | | | | |
| Raw material | Raw material 1 | Raw material 1 | Raw materia l 1 | Raw material 1 | Raw material 1 |
| Catalyst | Cat-F1 | Cat-F2 | Cat-F3 | Cat-F4 | Cat-B1 |
| Reaction temperature/°C | 400 | 400 | 400 | 400 | 395 |
| Reaction pressure/MPa | 4 | 4 | 4 | 4 | 3.0 |
| Volume ratio of hydrogen to oil | 600 | 600 | 600 | 600 | 400 |
| Volume space velocity/h-1 | 1.2 | 1.2 | 1.2 | 1.2 | 1.0 |
| Content of product C2-C6 n-alkanes, wt% | 52 | 51 | 48 | 50 | 92 |

| Preparation of ethylene by steam cracking | | | | | |
|---|---|---|---|---|---|
| Reaction temperature/°C | 880 | 880 | 880 | 880 | 880 |
| Reaction pressure/MPa | 0.3 | 0.3 | 0.3 | 0.3 | 0.3 |
| Mass ratio of water to oil | 0.4 | 0.4 | 0.4 | 0.4 | 0.4 |
| Yield of ethylene, wt% | 34.2 | 33.7 | 32.6 | 33.2 | 47.0 |
| Yield of propylene, wt% | 17.3 | 17.4 | 17.7 | 17.5 | 9.8 |
| Yield of butadiene, wt% | 3.7 | 3.7 | 3.8 | 3.8 | 4.0 |
| Processing benefit per ton oil, CNY/ton | Reference +170 | Referenc e+170 | Refere nce+19 0 | Reference +180 | Reference + 160 |

| No. | Example 22 | Example 23 | | | |
|---|---|---|---|---|---|
| Hydroconversion | | | | | |
| Raw material | Raw material 1 | Raw material 1 | | | |
| Catalyst | Cat-B2 | Cat-B3 | | | |
| Reaction temperature/°C | 393 | 398 | | | |
| Reaction pressure/MPa | 3.0 | 3.0 | | | |
| Volume ratio of hydrogen to oil | 400 | 400 | | | |
| Volume space velocity/h-1 | 1.0 | 1.0 | | | |
| Content of product C2-C6 n-alkanes, wt% | 90 | 89 | | | |
| Preparation of ethylene by steam cracking | | | | | |
| Reaction temperature/°C | 880 | 880 | | | |
| Reaction pressure/MPa | 0.3 | 0.3 | | | |
| Mass ratio of water to oil | 0.4 | 0.4 | | | |
| Yield of ethylene, wt% | 45.8 | 45.3 | | | |
| Yield of propylene, wt% | 10.2 | 10.5 | | | |
| Yield of butadiene, wt% | 4.1 | 4.1 | | | |
| Processing benefit per ton oil, CNY/ton | Reference + 60 | Referenc e+ 80 | | | |

**Continued Table 3 Conversion results**

| No. | Comparative Example 1 | Comparative Example 2 | Comparative Example 3 | Comparative Example 4 |
|---|---|---|---|---|
| Hydroconversion | | | | |
| Raw material | Raw material 4 | Raw material 4 | Raw material 5 | Raw material 5 |
| Catalyst | Cat-A1 | Cat-A4 | Cat-A1 | Cat-A4 |
| Reaction temperature/°C | 400 | 420 | 400 | 420 |
| Reaction pressure/MPa | 4 | 4 | 4 | 4 |
| Volume ratio of hydrogen to oil | 600 | 500 | 600 | 500 |
| Volume space velocity/h⁻¹ | 1.2 | 0.8 | 1.2 | 0.8 |
| Content of C₂-C₆ n-alkanes in the product, wt% | 45 | 75 | 43 | 72 |

| Preparation of ethylene by steam cracking | | | | |
|---|---|---|---|---|
| Reaction temperature/°C | 880 | 880 | 880 | 880 |
| Reaction pressure/MPa | 0.3 | 0.3 | 0.3 | 0.3 |
| Mass ratio of water to oil | 0.4 | 0.4 | 0.4 | 0.4 |
| Yield of ethylene, wt% | 31.7 | 39.8 | 30.8 | 38.9 |
| Yield of propylene, wt% | 18.6 | 13.7 | 18.7 | 14 |
| Yield of butadiene, wt% | 3.9 | 3.4 | 4 | 3.5 |
| Processing benefit per ton oil, CNY/ton | Reference-30 | Reference-50 | Reference-50 | Reference-70 |

| No. | Comparative Example 5 | Comparative Example 6 | Comparative Example 7 | Comparative Example 8 |
|---|---|---|---|---|
| Hydroconversion | | | | |
| Raw material | Raw material 1 | Raw material 6 | Raw material 1 | Raw material 1 |
| Catalyst | Cat-A7 | Cat-A1 | Cat-E | Cat-A4 |
| Reaction temperature/°C | 330 | 410 | 420 | 400 |
| Reaction pressure/MPa | 4 | 4 | 4 | 4 |
| Volume ratio of hydrogen to oil | 600 | 400 | 400 | 400 |
| Volume space velocity/h⁻¹ | 1 | 0.6 | 0.6 | 0.8 |
| Content of C₂-C₆ n-alkanes in the product, wt% | 44 | 36 | 24 | 19 |

| Preparation of ethylene by steam cracking | | | | |
|---|---|---|---|---|
| Reaction temperature/°C | 880 | 880 | 880 | 880 |
| Reaction pressure/MPa | 0.3 | 0.3 | 0.3 | 0.3 |
| Mass ratio of water to oil | 0.4 | 0.4 | 0.4 | 0.4 |
| Yield of ethylene, wt% | 31.6 | 28.4 | 26.5 | 24.5 |
| Yield of propylene, wt% | 18.5 | 20.1 | 20.3 | 19.3 |
| Yield of butadiene, wt% | 3.8 | 4.1 | 4.3 | 3.3 |
| Processing benefit per ton oil, CNY/ton | Reference-50 | Reference-40 | Reference-100 | Reference-150 |

| No. | Comparative Example 9 | Comparative Example 10 | Comparative Example 11 | Comparative Example 12 |
|---|---|---|---|---|
| Hydroconversion | | | | |
| Raw material | Raw material 1 | Raw material 1 | Raw material 1 | Raw material 1 |
| Catalyst | Cat-A1 | Cat-A1 | Cat-A1 | Cat-A1 |
| Reaction temperature/°C | 250 | 200 | 400 | 400 |
| Reaction pressure/MPa | 4 | 4 | 4 | 4 |
| Volume ratio of hydrogen to oil | 600 | 600 | 20 | 5 |
| Volume space velocity/h⁻¹ | 1.2 | 1.2 | 1.2 | 1.2 |
| Content of C₂-C₆ n-alkanes in the product, wt% | 49 | 49 | 49 | 49 |

| Preparation of ethylene by steam cracking | | | | |
|---|---|---|---|---|
| Reaction temperature/°C | 880 | 880 | 880 | 880 |
| Reaction pressure/MPa | 0.3 | 0.3 | 0.3 | 0.3 |
| Mass ratio of water to oil | 0.4 | 0.4 | 0.4 | 0.4 |
| Yield of ethylene, wt% | 26.6 | 26.6 | 29.2 | 28.5 |
| Yield of propylene, wt% | 19.1 | 19.1 | 18.7 | 18.8 |
| Yield of butadiene, wt% | 3.3 | 3.3 | 3.2 | 3.2 |
| Processing benefit per ton oil, CNY/ton | -120 | -120 | -110 | -100 |

| No. | Comparative Example 13 | Comparative Example 14 | Comparative Example 15 | |
|---|---|---|---|---|
| Hydroconversion | | | | |
| Raw material | Raw material 1 | Raw material 1 | Raw material 1 | |
| Catalyst | Solid acid catalyst | Cat-G | Cat-A1 | |
| Reaction temperature/°C | 400 | 415 | 400 | |
| Reaction pressure/MPa | 3.0 | 4 | 4 | |
| Volume ratio of hydrogen to oil | 600 | 800 | 600 | |
| Volume space velocity/h⁻¹ | 1.2 | 1.0 | 1.2 | |
| Content of C₂-C₆ n-alkanes in the product, wt% | 41 | 19 | 47 | |

| Preparation of ethylene by steam cracking | | | | |
|---|---|---|---|---|
| Reaction temperature/°C | 880 | 880 | 880 | |
| Reaction pressure/MPa | 0.3 | 0.3 | 0.3 | |
| Mass ratio of water to oil | 0.4 | 0.4 | 0.4 | |
| Yield of ethylene, wt% | 30.1 | 24.5 | 32.1 | |
| Yield of propylene, wt% | 19.0 | 19.3 | 17.8 | |
| Yield of butadiene, wt% | 3.9 | 3.3 | 3.8 | |
| Processing benefit per ton oil, CNY/ton | -30 | Reference-150 | Reference-20 | |

By comparison, the process according to the present invention could realize selective conversion of iso-alkanes, could flexibly adjust the ratio of C₂-C₃ hydrocarbons to C₄-C₆ n-alkanes in the converted product, in turn realized flexible adjustment of the structure of the ethylene product and improved the flexibility of the ethylene device product.

### Third series of Examples

In the following Examples and Comparative Examples, the yield was calculated as the mass percent of the output compared to the feed amount, and the hydrogen consumption was calculated as the mass percent of the hydrogen consumption compared to the feed amount.

In the following Examples and Comparative Examples, each hydroconversion catalyst was represented by Cat-A, Cat-B, Cat-C. The hydroconversion catalyst was prepared by a conventional saturation impregnation process for active metal, and the physicochemical properties of the obtained catalyst were shown in Table 1.

The catalyst of Cat-A was prepared by the process comprising:
(1) selecting mordenite with a SiO₂/Al₂O₃ molar ratio of 15, mechanically mixing the mordenite with a binder, molding, drying at 120°C for 10 hours, and calcinating at 500°C for 4 hours, to prepare a catalyst support.
(2) impregnating the catalyst support obtained in step (1) with a solution containing active components, drying at 120°C for 10 hours, and calcinating at 500°C for 4 hours, to prepare the hydroconversion catalyst.

The catalyst of Cat-B was prepared by the process comprising:
(1) selecting ZSM-5 molecular sieve with a SiO₂/Al₂O₃ molar ratio of 10, mechanically mixing the ZSM-5 with a binder, molding, drying at 120°C for 10 hours, and calcinating at 500°C for 4 hours, to prepare a catalyst support.
(2) impregnating the catalyst support obtained in step (1) with a solution containing active components, drying at 120°C for 10 hours, and calcinating at 500°C for 4 hours, to prepare the hydroconversion catalyst.

The catalyst of Cat-C was prepared by the process comprising:
(1) selecting beta molecular sieve with a SiO₂/Al₂O₃ molar ratio of 30, mechanically mixing the beta molecular sieve with a binder, molding, drying at 120°C for 10 hours, and calcinating at 500°C for 4 hours, to prepare a catalyst support.
(2) impregnating the catalyst support obtained in step (1) with a solution containing active components, drying at 120°C for 10 hours, and calcinating at 500°C for 4 hours, to prepare the hydroconversion catalyst.

The catalyst of Cat-D was prepared by the process comprising:
(1) selecting Y molecular sieve with a SiO₂/Al₂O₃ molar ratio of 15, mechanically mixing the Y molecular sieve with a binder, molding, drying at 120°C for 10 hours, and calcinating at 500°C for 4 hours, to prepare a catalyst support.
(2) impregnating the catalyst support obtained in step (1) with a solution containing active components, drying at 120°C for 10 hours, and calcinating at 500°C for 4 hours, to prepare the hydroconversion catalyst.

**Table 1**

| Catalyst | Cat-A | Cat-B | Cat-C | Cat-D |
|---|---|---|---|---|
| Pore volume, cm³/g | 0.35 | 0.35 | 0.35 | 0.35 |
| Specific surface area, m²/g | 300 | 300 | 300 | 300 |

| Content, wt%, based on the weight of the support | | | | |
|---|---|---|---|---|
| Mordenite | 55 | | | |
| ZSM-5 | | 55 | | |
| Beta | | | 55 | |
| Y | | | | 55 |
| Alumina | 19 | 19 | 19 | 19 |

| Active metal content in the catalyst, wt% | | | | |
|---|---|---|---|---|
| MoO₃ | 20 | 20 | 20 | 20 |
| NiO | 6 | 6 | 6 | 6 |

**Table 2**

| Raw material | | Raw material 1 |
|---|---|---|
| | C₄ iso-alkane content, wt% | 1 |
| | C₄ n-alkane content, wt% | 2 |
| | C₃-C₆ n-alkanes content, wt% | 16 |
| | C₃-C₆ iso-alkanes content, wt% | 78 |
| | C₇₊ Hydrocarbon and Cyclic Hydrocarbon content, wt% | 3 |
| Initial boiling point, °C | | 24 |
| End boiling point, °C | | 64 |

### Example 1

The procedures according to Fig.1 were used for the chemical conversion process, comprising:
(1) the raw material 1 and hydrogen were mixed and sequentially fed into a hydroconversion reaction zone; which hydroconversion reaction zone was loaded with hydroconversion catalyst Cat-A;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, the hydrogenated product had a content of C₅-C₆ n-alkanes of 20.2%, and a content be C₂-C₃ hydrocarbons of 23%.

### Example 2

The procedures according to Fig.1 were used for the chemical conversion process, comprising:
(1) the raw material 1 and hydrogen were mixed and sequentially fed into a hydroconversion reaction zone; which hydroconversion reaction zone was loaded with hydroconversion catalyst Cat-A;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, the hydrogenated product had a content of C₅-C₆ n-alkanes of 16.6%, and a content be C₂-C₃ hydrocarbons of 30%.

### Example 3

The procedures according to Fig.1 were used for the chemical conversion process, comprising:
(1) the raw material 1 and hydrogen were mixed and sequentially fed into a hydroconversion reaction zone; which hydroconversion reaction zone was loaded with hydroconversion catalyst Cat-A;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, the hydrogenated product had a content of C₅-C₆ n-alkanes of 11.1%, and a content be C₂-C₃ hydrocarbons of 41.5%.

### Comparative Example 1

The procedures according to Fig.1 were used for the chemical conversion process, comprising:
(1) the raw material 1 and hydrogen were mixed and sequentially fed into a hydroconversion reaction zone; which hydroconversion reaction zone was loaded with hydroconversion catalyst Cat-B;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, the hydrogenated product had a content of C₅-C₆ n-alkanes of 2.2%, and a content be C₂-C₃ hydrocarbons of 60.4%.

### Comparative Example 2

The procedures according to Fig.1 were used for the chemical conversion process, comprising:
(1) the raw material 1 and hydrogen were mixed and sequentially fed into a hydroconversion reaction zone; which hydroconversion reaction zone was loaded with hydroconversion catalyst Cat-C;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, the hydrogenated product had a content of C₅-C₆ n-alkanes of 17.2%, and a content be C₂-C₃ hydrocarbons of 14.4%.

### Comparative Example 3

The procedures according to Fig.1 were used for the chemical conversion process, comprising:
(1) the raw material 1 and hydrogen were mixed and sequentially fed into a hydroconversion reaction zone; which hydroconversion reaction zone was loaded with hydroconversion catalyst Cat-D;
(2) the reaction effluent from the hydroconversion reaction zone of step (1) was subjected to gas-liquid separation, to obtain a hydrogen-rich gas and a liquid-phase stream, recycling the hydrogen-rich gas, and directly using the liquid-phase stream as an ethylene raw material. The process conditions and the conversion results in this Example were shown in Table 3.

In this Example, the hydrogenated product had a content of C₅-C₆ n-alkanes of 15.1%, and a content be C₂-C₃ hydrocarbons of 10.4%.

**Table 3**

| No. | Example 1 | Example 2 | Example 3 |
|---|---|---|---|
| Hydroconversion | | | |
| Raw material | Raw material 1 | Raw material 1 | Raw material 1 |
| Catalyst | Cat-A | Cat-A | Cat-A |
| Reaction temperature/°C | 380 | 390 | 400 |
| Reaction pressure/MPa | 4 | 4 | 4 |
| Volume ratio of hydrogen to oil | 600 | 600 | 600 |
| Volume space velocity/h⁻¹ | 1.0 | 1.0 | 1.0 |
| Content of C₂-C₆ n-alkanes in the product, wt% | 50 | 53 | 62 |
| Hydrogen consumption, % | 0.77 | 0.99 | 1.43 |

| Preparation of ethylene by steam cracking | | | |
|---|---|---|---|
| Reaction temperature/°C | 880 | 880 | 880 |
| Reaction pressure/MPa | 0.3 | 0.3 | 0.3 |
| Mass ratio of water to oil | 0.4 | 0.4 | 0.4 |
| Yield of ethylene, wt% | 33.2 | 33.8 | 35.7 |
| Yield of propylene, wt% | 17.6 | 17.4 | 16.5 |
| Yield of butadiene, wt% | 3.8 | 3.7 | 3.5 |

**Continued Table 3**

| No. | Comparative Example 1 | Comparative Example 2 | Comparative Example 3 |
|---|---|---|---|
| Hydroconversion | | | |
| Raw material | Raw material 1 | Raw material 1 | Raw material 1 |
| Catalyst | Cat-B | Cat-C | Cat-D |
| Reaction temperature/°C | 400 | 400 | 400 |
| Reaction pressure/MPa | 4 | 4 | 4 |
| Volume ratio of hydrogen to oil | 600 | 600 | 600 |
| Volume space velocity/h⁻¹ | 1.0 | 1.0 | 1.0 |
| Content of C₂-C₆ n-alkanes in the product, wt% | 69 | 36 | 28 |
| Hydrogen consumption, % | 2.12 | 0.42 | 0.35 |

| Preparation of ethylene by steam cracking | | | |
|---|---|---|---|
| Reaction temperature/°C | 880 | 880 | 880 |
| Reaction pressure/MPa | 0.3 | 0.3 | 0.3 |
| Mass ratio of water to oil | 0.4 | 0.4 | 0.4 |
| Yield of ethylene, wt% | 36.8 | 30.2 | 27.8 |
| Yield of propylene, wt% | 15.2 | 19.8 | 21.3 |
| Yield of butadiene, wt% | 3.0 | 4.1 | 4.3 |

## Claims

1. A process for chemical conversion of a hydrocarbon stream, comprising the steps of:
1) providing a hydrocarbon stream comprising a paraffinic alkane represented by the structural formula (I) of: 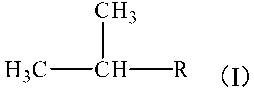 in which formula (I), R is a C₂-C₆ linear or branched alkyl (preferably a C₂-C₄ linear or branched alkyl, more preferably a C₂-C₃ linear alkyl),
2) converting at least a part (such as 30wt% or more, 40wt% or more, 50wt% or more, 60wt% or more, 70wt% or more, 80wt% or more or 90wt% or more of the total amount) of the paraffinic alkane in a conversion reaction to a C₂-C₆ n-alkane in the presence of a catalyst and hydrogen gas to obtain a converted product, wherein the catalyst comprises a molecular sieve and a non-noble metal hydrogenation-active metal component,
3) steam cracking the converted product to obtain a cracked product comprising ethylene.

2. The process according to claim 1, wherein the molecular sieve is one or more selected from the group consisting of mordenite, ZSM molecular sieve, SAPO molecular sieve and EU-1 molecular sieve, more preferably one or more selected from the group consisting of mordenite and ZSM molecular sieve, especially preferably one or more selected from the group consisting of mordenite, ZSM-5 molecular sieve, ZSM-11 molecular sieve, ZSM-12 molecular sieve, ZSM-22 molecular sieve, ZSM-23 molecular sieve, ZSM-35 molecular sieve, Beta molecular sieve and ZSM-38 molecular sieve, especially one or more selected from the group consisting of mordenite and ZSM-5 molecular sieve, preferably the weight ratio of mordenite to ZSM-5 molecular sieve is in the range of 0-100: 100-0 (preferably 10-50: 50-10).

3. The process according to claim 1, wherein the non-noble metal hydrogenation active metal component is one or more selected from the group consisting of a non-noble metal of Group VIB, a non-noble metal of Group VIII, an oxide thereof or a sulfide thereof, more preferably one or more selected from the group consisting of molybdenum, tungsten, cobalt, nickel, a sulfide thereof or an oxide thereof.

4. The process according to claim 3, wherein the non-noble metal of Group VIB (calculated as oxide) is present in an amount of 5.0-30.0 wt% (preferably 10-20 wt%) and the non-noble metal of Group VIII (calculated as oxide) is present in an amount of 0.5-15.0 wt% (preferably 3-10 wt%), based on the weight of the catalyst.

5. The process according to claim 1, wherein the molecular sieve is present in an amount of 30-80 wt% (preferably 40-70 wt%), based on the weight of the catalyst.

6. The process according to claim 1, wherein the conversion reaction is carried out under the reaction conditions of: a reaction pressure of 0.5-10.0 MPaG (preferably 2.0-8.0 MPaG or 2.0-5.0 MPaG), a reaction temperature of 300-500 °C (preferably 350-450 °C), a liquid hourly space velocity of 0.1-15.0 h⁻¹ (preferably 0.5-5.0 h⁻¹), and a volume ratio of hydrogen to oil of 50:1-2500:1 (preferably 100:1-2000:1 or 100:1-1000:1).

7. The process according to claim 1, wherein in the hydrocarbon stream, C₇+ hydrocarbons are present in an amount of 0-10wt% (preferably 0.5-5 wt%), C₅₋₆ iso-alkanes are present in an amount of 50 wt% or more (preferably 60-90wt%, more preferably 70-80 wt%), C₅-C₆ n-alkanes are present in an amount of 10-30wt% (preferably 15-25 wt%), C₄- hydrocarbons are present in an amount of 0-10wt% (preferably 2-5 wt%), and cyclic hydrocarbons are present in an amount of 1 to 10wt% (preferably 2-5 wt%), based on the total weight of the hydrocarbon stream being 100 wt%.

8. The process according to claim 1, wherein the converted product comprises C₂-C₃ alkanes and C₄-C₆ n-alkanes (preferably C₅-C₆ n-alkanes), and the weight ratio of the C₂-C₃ alkanes to the C₄-C₆ n-alkanes (preferably C₅-C₆ n-alkanes) is 0.5:1-8:1 (preferably 0.9:1-5:1).

9. The process according to claim 1, wherein in the converted product, C₇+ hydrocarbons are present in an amount of not more than 5wt% (preferably not more than 1 wt%), C₅-C₆ iso-alkanes are present in an amount of 0-50 wt% (preferably 10-40 wt%), C₂-C₆ n-alkanes are present in an amount of 40-90wt% (preferably 50-80 wt%), cyclic hydrocarbons are present in an amount of not more than 3wt% (preferably not more than 1 wt%), based on the total weight of the converted product being 100 wt%.

10. The process according to claim 1, wherein the step 3) comprises the steps of:
3-A-1) separating the converted product (called as a first separation) to obtain a separated stream with C₂+ hydrocarbons as the main part (such as representing 95 wt% or more, 98 wt% or more, 99 wt% or more, or essentially 100 wt% of the total amount),
3-A-2) steam cracking the separated stream to obtain a cracked product comprising ethylene.

11. The process according to claim 10, wherein the first separation is operated under conditions comprising a pressure of 0.5-10.0 MPaG (preferably 2.0-8.0 MPaG or 2.0-5.0 MPaG) and a temperature of 40-70 °C.

12. The process according to claim 1, wherein the step 3) comprises the steps of:
3-B-1) separating the converted product (called as a first separation) to obtain a separated stream with C₂+ hydrocarbons as the main part (such as representing 95 wt% or more, 98 wt% or more, 99 wt% or more, or essentially 100 wt% of the total amount),
3-B-2) separating the separated stream (called as a second separation) to obtain a high-carbon stream comprising C₄⁺ hydrocarbons as the main part (such as 90 wt% or more, 95 wt% or more, 98 wt% or more, 99 wt% or more, or substantially 100 wt% of the total amount) and a low-carbon stream comprising C₂-C₃ hydrocarbons as the main part (such as 90 wt% or more, 95 wt% or more, 98 wt% or more, 99 wt% or more, or substantially 100 wt% of the total amount),
3-B-3) optionally recycling the high-carbon stream as a hydrocarbon stream back to the step 2) for the conversion reaction,
3-B-4) steam cracking the low-carbon stream to obtain a cracked product comprising ethylene.

13. The process according to claim 12, wherein the first separation is operated under conditions comprising a pressure of 0.5-10.0 MPaG (preferably 2.0-8.0 MPaG or 2.0-5.0 MPaG) and a temperature of 40-70 °C, and the second separation is operated under conditions comprising a pressure of 0.5-1.5 MPaG and a temperature of 30-60 °C.

14. A hydrocarbon mixture comprising C₅-C₆ n-alkanes and C₂-C₃ alkanes, wherein the C₅-C₆ n-alkanes are present in an amount of 5-30 wt% (preferably 10-25 wt%) and the C₂-C₃ alkanes are present in an amount of 20-50 wt% (preferably 25-45 wt%), based on 100wt% of the total weight of the hydrocarbon mixture.
